# EUROPÄISCHE PATENTANMELDUNG

(11) **EP 4 443 703 A1**
(43) Veröffentlichungstag der Anmeldung: **09.10.2024**
(21) Anmeldenummer: 24165046.4
(22) Anmeldetag: 21.03.2024
(51) Int. Cl.: H02K 1/14, A61M 60/232, H02K 3/24, H02K 5/128, H02K 7/09, H02K 9/22, H02K 11/33, A61M 60/109, A61M 60/422, F04D 13/06, H02K 9/197

(54) **ELEKTROMAGNETISCHER DREHANTRIEB UND ZENTRIFUGALPUMPE**

(30) Priorität: 03.04.2023 EP 23166383
(71) Anmelder: Levitronix GmbH, 8048 Zürich (CH)
(72) Erfinder: Willi, Urs, 8590 Romanshorn (CH); Häfliger, Mario, 5625 Kallern (CH); Barletta, Natale, 8048 Zürich (CH)
(74) Vertreter: IPS Irsch AG

(57) **Zusammenfassung**

Es wird ein elektromagnetischer Drehantrieb vorgeschlagen, der als Tempelmotor ausgestaltet ist, mit einem Rotor (3), der einen ring- oder scheibenförmigen magnetisch wirksamen Kern (31) umfasst, sowie mit einem Stator (2), welcher als Lager- und Antriebsstator ausgestaltet ist, mit welchem der Rotor (3) im Betriebszustand berührungslos magnetisch um eine Solldrehachse antreibbar ist, die eine axiale Richtung (A) definiert, und mit welchem der Rotor (3) berührungslos magnetisch bezüglich des Stators (2) lagerbar ist, wobei der Rotor (3) in einer zur axialen Richtung (A) senkrechten radialen Ebene (E) aktiv magnetisch gelagert ist, wobei der Stator (2) eine Mehrzahl von Spulenkernen (25) aufweist, von denen jeder einen Längsschenkel (26) umfasst, welcher sich von einem ersten Ende in axialer Richtung (A) bis zu einem zweiten Ende erstreckt, sowie einen Querschenkel (27), welcher an dem zweiten Ende des Längsschenkels (26) und in der radialen Ebene (E) angeordnet ist, und welcher sich von dem Längsschenkel (26) in einer radialen Richtung erstreckt, wobei die Spulenkerne (25) bezüglich einer Umfangsrichtung um den magnetisch wirksamen Kern (31) herum angeordnet sind, und wobei an jedem Längsschenkel (26) mindestens eine konzentrierte Wicklung (61; 61a, 61b) vorgesehen ist, welche den jeweiligen Längsschenkel (26) umgibt. Es ist eine Kühlvorrichtung (10) vorgesehen, welche eine erste Kühlleitung (8) umfasst, die von einem Kühlfluid durchströmbar ist, wobei die erste Kühlleitung (8) mindestens einen Kühlleitungsabschnitt (80) aufweist, welcher bezüglich der Umfangsrichtung in einem Zwischenraum zwischen den Längsschenkeln (26) zweier benachbarter Spulenkerne (25) angeordnet ist, und welcher sich in der axialen Richtung (A) erstreckt. Durch die Erfindung werden ferner eine Zentrifugalpumpe mit einem solchen Drehantrieb (1) vorgeschlagen.

## Beschreibung

Die Erfindung betrifft einen elektromagnetischen Drehantrieb gemäss dem Oberbegriff des unabhängigen Patentanspruchs und eine Zentrifugalpumpe mit einem solchen elektromagnetischen Drehantrieb.

Es sind elektromagnetische Drehantriebe bekannt, die nach dem Prinzip des lagerlosen Motors ausgestaltet sind und betrieben werden. Mit dem Begriff lagerloser Motor ist dabei ein elektromagnetischer Drehantrieb gemeint, bei welchem der Rotor vollkommen magnetisch bezüglich des Stators gelagert ist, wobei keine separaten magnetischen Lager vorgesehen sind. Der Stator ist dazu als Lager- und Antriebsstator ausgestaltet, der sowohl Stator des elektrischen Antriebs als auch Stator der magnetischen Lagerung ist. Mit den elektrischen Wicklungen des Stators lässt sich ein magnetisches Drehfeld erzeugen, welches zum einen ein Drehmoment auf den Rotor ausübt, das dessen Rotation um eine Solldrehachse bewirkt, und welches zum anderen eine beliebig einstellbare Querkraft auf den Rotor ausübt, sodass dessen radiale Position aktiv steuerbar bzw. regelbar ist. Somit sind drei Freiheitsgrade des Rotors aktiv regelbar, nämlich seine Rotation sowie seine radiale Position (zwei Freiheitsgrade). Bezüglich dreier weiterer Freiheitsgrade, nämlich seiner Position in axialer Richtung und Verkippungen bezüglich der zur Solldrehachse senkrechten radialen Ebene (zwei Freiheitsgrade), ist der Rotor passiv magnetisch, das heisst nicht ansteuerbar, durch Reluktanzkräfte gelagert bzw. stabilisiert. Das Nichtvorhandensein eines separaten magnetischen Lagers bei vollständiger magnetischer Lagerung des Rotors ist die Eigenschaft, welcher der lagerlose Motor seinen Namen verdankt. In dem Lager- und Antriebsstator lässt sich die Lagerfunktion nicht von der Antriebsfunktion separieren.

Ein solcher lagerloser Motor hat sich in einer Vielzahl von Anwendungen bewährt. Aufgrund der Abwesenheit von mechanischen Lagern eignet sich der lagerlose Motor insbesondere für Pump-, Misch- oder Rührvorrichtungen, mit denen sehr empfindliche Substanzen gefördert werden, beispielsweise Blutpumpen, oder bei denen sehr hohe Anforderungen an die Reinheit gestellt werden, beispielsweise in der pharmazeutischen Industrie oder in der biotechnologischen Industrie, oder mit denen abrasive oder aggressive Substanzen gefördert werden, welche mechanische Lager sehr schnell zerstören würden, beispielsweise Pumpen oder Mischer für Slurry, Schwefel-, Phosphorsäure oder andere Chemikalien in der Halbleiterindustrie.

Ein weiterer Vorteil des Prinzips des lagerlosen Motors ergibt sich bei der Ausgestaltung des Rotors als Integralrotor, der sowohl der Rotor des elektromagnetischen Drehantriebs ist als auch der Rotor der Pumpe. Neben der berührungslosen magnetischen Lagerung resultiert hier der Vorteil einer sehr kompakten und platzsparenden Ausgestaltung.

Zudem erlaubt das Prinzip des lagerlosen Motors auch Ausgestaltungen, beispielsweise von Zentrifugalpumpen, bei denen der Rotor sehr leicht vom Stator trennbar ist. Dies ist ein sehr grosser Vorteil, weil damit beispielsweise der Rotor bzw. die Pumpeneinheit, welche den Rotor umfasst, als Einmalteil für den Einmalgebrauch ausgestaltet werden kann. Solche Einmalanwendungen ersetzen heute häufig Prozesse, bei denen früher aufgrund der sehr hohen Reinheitsanforderungen alle diejenigen Komponenten, welche im Prozess mit den zu behandelnden Substanzen in Kontakt kommen, aufwändig gereinigt und sterilisiert werden müssen, beispielsweise mittels Dampfsterilisierung. Bei der Ausgestaltung für den Einmalgebrauch werden diejenigen Komponenten, welche mit den zu behandelnden Substanzen in Kontakt kommen, nur genau einmal verwendet und dann bei der nächsten Anwendung durch neue, das heisst ungebrauchte, Einmalteile ersetzt.

Als Beispiele seien hier die Pharmaindustrie und die biotechnologische Industrie genannt. Hier werden häufig Lösungen und Suspensionen hergestellt, die eine sorgfältige und schonende Förderung von Substanzen verlangen.

Eine vorteilhafte und an sich bekannte Ausführungsform eines elektromagnetischen Drehantriebs, der nach dem Prinzip des lagerlosen Motors ausgestaltet werden kann, ist die Ausführung als Tempelmotor, die beispielsweise in der EP-A-3 232 549 offenbart wird. Auf die Ausgestaltung als Tempelmotor bezieht sich auch die vorliegende Erfindung.

Das Charakteristische eines Tempelmotors ist es, dass der Stator eine Mehrzahl von Spulenkernen aufweist, von denen jeder einen Längsschenkel umfasst, der sich parallel zur axialen Richtung erstreckt. Mit der axialen Richtung ist dabei diejenige Richtung gemeint, welche durch die Solldrehachse des Rotors definiert ist, also die Drehachse, um welche der Rotor im Betriebszustand rotiert, wenn er in der radialen Ebene, die senkrecht zur axialen Richtung liegt, bezüglich des Stators in einer zentrierten und unverkippten Position ist. Jeder Längsschenkel erstreckt sich von einem ersten Ende in axialer Richtung bis zu einem zweiten Ende. Jeder Spulenkern umfasst zusätzlich zu dem Längsschenkel einen Querschenkel, welcher jeweils an dem zweiten Ende des Längsschenkels vorgesehen ist, und welcher sich in radialer Richtung nach innen erstreckt, also im Wesentlichen rechtwinklig zum Längsschenkel. Die Spulenkerne haben jeweils die Form eines L, wobei die Querschenkel die kurzen Schenkel des L bilden. Der Rotor ist dann zwischen den Querschenkeln angeordnet.

Die Mehrzahl der Längsschenkel, die sich in axialer Richtung erstrecken und an die Säulen eines Tempels erinnern, hat dem Tempelmotor seinen Namen gegeben.

In einer Ausführungsform hat der Tempelmotors beispielsweise sechs Spulenkerne, die kreisförmig und äquidistant um den Rotor herum angeordnet sind (Innenläufer). Die ersten Enden der Längsschenkel sind in Umfangsichtung durch einen Rückschluss verbunden, welcher der magnetischen Flussführung dient. Der Rotor umfasst einen magnetisch wirksamen Kern, beispielsweise eine permanentmagnetische Scheibe oder einen permanentmagnetischen Ring, der zwischen den radial innenliegenden Enden der Querschenkel angeordnet ist und im Betriebszustand um die axiale Richtung rotiert, wobei der Rotor berührungslos magnetisch angetrieben und berührungslos magnetisch bezüglich des Stators gelagert ist.

Es sind auch solche Ausgestaltungen des Tempelmotors bekannt, bei denen der magnetisch wirksame Kern permanentmagnetfrei, also ohne Permanentmagnete ausgestaltet ist. Der magnetisch wirksame Kern des Rotors ist dann beispielsweise ferromagnetisch ausgestaltet und besteht beispielsweise aus Eisen, Nickel-Eisen, Kobalt-Eisen, Silizium-Eisen, Mu-Metall oder einem anderen ferromagnetischen Werkstoff.

Ferner sind Ausgestaltungen möglich, bei denen der magnetisch wirksame Kern des Rotors sowohl ferromagnetische Materialien als auch permanentmagnetische Materialien umfasst. Beispielsweise können Permanentmagnete in einen ferromagnetischen Grundkörper eingelegt bzw. eingesetzt werden. Solche Ausgestaltungen sind z.B. vorteilhaft, wenn man bei grossen Rotoren die Kosten durch Einsparen von permanentmagnetischem Material reduzieren will.

Um die für den magnetischen Antrieb und die magnetische Lagerung des Rotors notwendigen elektromagnetischen Drehfelder zu erzeugen, tragen die Längsschenkel Wicklungen. Die Wicklungen sind beispielsweise so ausgestaltet, dass um jeden Längsschenkel herum eine konzentrierte Wicklung gewickelt ist, das heisst, die Spulenachse jeder konzentrierten Wicklung erstreckt sich jeweils in axialer Richtung. Dabei ist es typisch für den Tempelmotor, dass die Spulenachsen der konzentrierten Wicklungen parallel zur Solldrehachse verlaufen und, dass die konzentrierten Wicklungen nicht in der radialen Ebene angeordnet sind, in welcher der Rotor bzw. der magnetisch wirksame Kern des Rotors im Betriebszustand gelagert wird.

Ein Problem beim Betrieb solcher elektromagnetischer Drehantriebe ist die Abführung der im Betrieb generierten Wärme, beispielsweise der durch die Eisen- und Kupferverluste erzeugten Wärme. Insbesondere für die elektronischen Komponenten des Drehantriebs führt eine erhöhte Temperatur zu einer Verringerung der Lebenddauer. Auch kann die Motorleistung durch das Abführen der Verlustwärme erhöht werden. Das Problem der Wärmeabführung verstärkt sich noch, wenn beispielsweise mit dem Drehantrieb Fluide mit sehr hoher Temperatur gefördert oder gemischt werden. Dann wird nämlich zusätzlich von dem Fluid noch Wärme in den elektromagnetischen Drehantrieb eingetragen. In der Halbleiterindustrie gibt es beispielsweise Anwendungen, bei denen das zu fördernde Fluid mehr als 200°C heiss ist, beispielsweise bis zu 220°C. Oft handelt es sich dabei zusätzlich um chemisch aggressive Substanzen, z.B. Schwefelsäure oder Phosphorsäure.

Um die Wärme, die durch den Betrieb generiert oder von dem Fluid in den Drehantrieb eingebracht wird, abzuführen, sind verschiedene Massnahmen bekannt. So kann beispielsweise das Gehäuse des Drehantriebs mit Kühlrippen versehen werden, welche die Wärme an die Umgebung abgeben.

Auch ist es bekannt, am Gehäuse des Drehantriebs einen Lüfter oder ein Gebläse vorzusehen, um den Drehantrieb mittels eines Luftstroms zu kühlen. Diese Lüfter haben allerdings eine begrenzte Lebensdauer und sind in der Regel chemisch wenig resistent, sodass sie beispielsweise für Anwendungen in der Halbleiterindustrie weniger geeignet sind.

Auch ist es bekannt, am Gehäuse des Drehantriebs eine Druckluftkühlung vorzusehen, beispielsweise mit einer an dem Gehäuse befestigten Haube, in welche Druckluft eingeblasen wird. Druckluft ist jedoch sehr teuer und daher im Hinblick auf die Wirtschaftlichkeit des Prozesses eher nachteilig.

Ferner ist es bekannt, am Gehäuse des Drehantriebs Kühlplatten anzubringen, die beispielsweise von einer Kühlflüssigkeit durchströmt werden, um die Wärme abzuführen. Es hat sich aber gezeigt, dass solche externen Kühlplatten häufig keine ausreichende Wärmeabfuhr gewährleisten. Ein Grund dafür sind die hohen thermischen Widerstände, beispielsweise an dem Gehäuse des Drehantriebs, sodass mittels der Kühlplatte nur in unzureichender Weise Wärme aus dem Inneren des Gehäuses bzw. den dort angeordneten Komponenten abgeführt werden kann.

In der Praxis hat es sich gezeigt, dass insbesondere bei Anwendungen, bei welchen das zu fördernde oder das zu mischende Fluid Temperaturen von mehr als 70°C hat, solche externen Kühlungen am Gehäuse des Drehantriebs keine zufriedenstellende Wärmeabfuhr gewährleisten.

Es sind auch solche Motoren bekannt, beispielsweise aus der US 10,530,221, bei welchen in der Gehäusewand des Motors Kühlstrukturen wie Bohrungen oder Kanäle vorgesehen sind, welche während des Betriebs des Motors von einer Kühlflüssigkeit durchströmt werden. Solche Kühlstrukturen in der Gehäusewand, die sehr aufwändig gefräst oder gebohrt werden müssen, sind sehr komplex und verursachen hohe Herstellungskosten. Zudem sind zusätzliche Abdichtungen notwendig. Oft müssen dann noch zusätzliche Beschichtungen vorgesehen werden, um die gewünschte chemische Resistenz zu gewährleisten. Dadurch werden zusätzliche thermische Widerstände generiert, welche die Kühlleistung wiederum reduzieren.

Ausgehend von diesem Stand der Technik ist es daher eine Aufgabe der Erfindung, einen elektromagnetischen Drehantrieb vorzuschlagen, der als Tempelmotor ausgestaltet ist, und bei welchem eine effiziente Wärmeabfuhr aus dem Drehantrieb gewährleistet ist. Im Speziellen soll der Drehantrieb auch für solche Anwendungen geeignet sein, bei welchen sehr heisse Fluide von beispielsweise bis zu 200°C oder noch mehr, gefördert oder gemischt werden. Ferner ist es eine Aufgabe der Erfindung, eine Zentrifugalpumpe mit einem solchen Drehantrieb vorzuschlagen.

Der diese Aufgabe lösende Gegenstand der Erfindung ist durch die Merkmale des unabhängigen Patentanspruchs gekennzeichnet.

Erfindungsgemäss wird also ein elektromagnetischer Drehantrieb vorgeschlagen, der als Tempelmotor ausgestaltet ist, mit einem Rotor, der einen ring- oder scheibenförmigen magnetisch wirksamen Kern umfasst, sowie mit einem Stator, welcher als Lager- und Antriebsstator ausgestaltet ist, mit welchem der Rotor im Betriebszustand berührungslos magnetisch um eine Solldrehachse antreibbar ist, die eine axiale Richtung definiert, und mit welchem der Rotor berührungslos magnetisch bezüglich des Stators lagerbar ist, wobei der Rotor in einer zur axialen Richtung senkrechten radialen Ebene aktiv magnetisch gelagert ist, wobei der Stator eine Mehrzahl von Spulenkernen aufweist, von denen jeder einen Längsschenkel umfasst, welcher sich von einem ersten Ende in axialer Richtung bis zu einem zweiten Ende erstreckt, sowie einen Querschenkel, welcher an dem zweiten Ende des Längsschenkels und in der radialen Ebene angeordnet ist, und welcher sich von dem Längsschenkel in einer radialen Richtung erstreckt, wobei die Spulenkerne bezüglich einer Umfangsrichtung um den magnetisch wirksamen Kern herum angeordnet sind, und wobei an jedem Längsschenkel mindestens eine konzentrierte Wicklung vorgesehen ist, welche den jeweiligen Längsschenkel umgibt. Es ist eine Kühlvorrichtung vorgesehen ist, welche eine erste Kühlleitung umfasst, die von einem Kühlfluid durchströmbar ist, wobei die erste Kühlleitung mindestens einen Kühlleitungsabschnitt aufweist, welcher bezüglich der Umfangsrichtung in einem Zwischenraum zwischen den Längsschenkeln zweier benachbarter Spulenkerne angeordnet ist, und welcher sich in der axialen Richtung erstreckt.

Dadurch, dass der mindestens eine Kühlleitungsabschnitt im Zwischenraum zwischen zwei benachbarten Längsschenkeln und damit auch zwischen zwei in Umfangsrichtung benachbarten Wicklungen angeordnet ist, wird die Wärme effizient von den Orten abgeführt, wo sie entsteht. Ein erheblicher Teil der im Betrieb generierten Wärme stammt von den Eisenverlusten, die beispielsweise in den Längsschenkeln auftreten, und von den Kupferverlusten, die in den Wicklungen auftreten. Da der Kühlleitungsabschnitt unmittelbar benachbart zu den Längsschenkeln und zu den darauf angeordneten Wicklungen angeordnet ist, kühlt er genau dort, wo zwei Hauptwärmequellen sind. Daher resultiert eine sehr effiziente Wärmeabfuhr aus dem Drehantrieb.

Typischerweise umfasst der Drehantrieb ein Statorgehäuse, in welchem der Stator angeordnet ist. Der Kühlleitungsabschnitt ist dann im Innenraum des Statorgehäuses angeordnet, sodass es nicht notwendig ist, komplizierte Kühlstrukturen, wie beispielsweise Bohrungen oder Ausfräsungen in der Wand des Statorgehäuses vorzusehen. Die Kühlvorrichtung kann daher problemlos in einen Tempelmotor integriert werden, ohne dass die Ausgestaltung der Komponenten des Tempelmotors wesentlich geändert werden muss. Dies ist unter konstruktiven Aspekten ein sehr grosser Vorteil.

Das Kühlfluid ist vorzugsweise eine Flüssigkeit, beispielsweise Wasser, destilliertes Wasser, oder bevorzugt demineralisiertes oder deionisiertes Wasser (DI Wasser).

Vorzugsweise ist der Kühlleitungsabschnitt Bestandteil einer Kühlschleife, die vollständig in einem der Zwischenräume zwischen den Längsschenkeln zweier benachbarte Längsschenkel angeordnet ist, oder die sich über mehrere dieser Zwischenräume erstreckt.

In einer bevorzugten Ausgestaltung weist die erste Kühlleitung mindestens eine Kühlschleife und vorzugsweise mehrere Kühlschleifen auf, wobei jede Kühlschleife bezüglich der Umfangsrichtung jeweils in einem Zwischenraum zwischen den Längsschenkeln zweier benachbarter Spulenkerne angeordnet ist, und wobei jeder Kühlleitungsabschnitt jeweils Bestandteil einer der Kühlschleifen ist. Bei einer Ausgestaltung der ersten Kühlleitung mit mehreren Kühlschleifen sind vorzugsweise Verbindungssegmente zum Verbinden benachbarter Kühlschleifen vorgesehen. Es sind Ausgestaltungen möglich, bei denen in jedem Zwischenraum zwischen zwei in Umfangsrichtung benachbarten Längsschenkeln jeweils eine Kühlschleife vorgesehen ist. Dann ist die Anzahl der Kühlschleifen vorzugsweise gleich der Anzahl der Spulenkerne.

Es sind aber auch solche Ausgestaltungen möglich, bei denen nicht in jedem Zwischenraum zwischen zwei in Umfangsrichtung benachbarten Längsschenkeln eine Kühlschleife vorgesehen ist. Die Anzahl der Kühlschleifen ist dann vorzugsweise kleiner als die Anzahl der Spulenkerne.

Besonders bevorzugt ist jede Kühlschleife jeweils U-förmig ausgestaltet, wobei sich die beiden Schenkel des U jeweils in axialer Richtung und somit parallel zu den Längsschenkeln der Spulenkerne erstrecken. Die geschlossene Seite des U ist näher an den zweiten Enden der Längsschenkel angeordnet als die offene Seite des U, welche näher an den ersten Enden der Längsschenkel angeordnet ist. Die Verbindungssegmente zwischen benachbarten Kühlschleifen verbinden jeweils einen Schenkel der U-förmigen Kühlschleife mit einem der Schenkel einer in Umfangsrichtung benachbarten U-förmigen Kühlschleife.

Vorzugsweise ist die erste Kühlleitung mit allen Kühlschleifen und mit allen Verbindungssegmenten einstückig ausgestaltet und erstreckt sich von einem ersten Kühlanschluss bis zu einem zweiten Kühlanschluss. Speziell bevorzugt wird die erste Kühlleitung durch Biegen eines Rohrs hergestellt. Beispielsweise wird ein ursprünglich gerades Stück Rohr durch Biegen derart geformt, dass die U-förmigen Kühlschleifen mit den dazwischen angeordneten Verbindungssegmenten entstehen.

Ferner ist es bevorzugt, wenn die erste Kühlleitung aus einem Edelstahl oder aus einem rostfreien Stahl besteht. Ein solcher Stahl hat auch den Vorteil, dass er korrosionsbeständiger ist als beispielsweise Kupfer. Insbesondere bei der Verwendung von DI Wasser als Kühlfluid, das aggressiver ist als beispielsweise normales Wasser, ist es vorteilhaft, die erste Kühlleitung aus einem rostfreien Stahl oder aus einem Edelstahl herzustellen.

Vorzugsweise weist also die erste Kühlleitung mehrere Kühlschleifen auf, sowie Verbindungssegmente zum Verbinden benachbarter Kühlschleifen, wobei sich die Verbindungssegmente bevorzugt jeweils in der Umfangsrichtung erstrecken, und radial aussenliegend benachbart zu den ersten Enden der Längsschenkel der Spulenkerne angeordnet sind.

In einer bevorzugten Ausführungsform sind die ersten Enden aller Längsschenkel mittels eines Rückschlusses zum Führen des magnetischen Flusses verbunden, wobei die erste Kühlleitung bereichsweise benachbart zu dem Rückschluss verläuft. Bereichsweise meint dabei insbesondere, dass sich die Verbindungssegmente entlang des Rückschlusses erstrecken können, sodass die im Rückschluss vorhandene Wärme, also insbesondere die durch Eisenverluste generierte Wärme, effizient dort aufgenommen und abgeführt wird, wo sie entsteht.

Es ist eine weitere bevorzugte Massnahme, dass die Kühlvorrichtung eine zweite Kühlleitung umfasst, die von dem Kühlfluid durchströmbar ist, wobei die zweite Kühlleitung eine Innenraumschleife aufweist, welche radial innenliegend bezüglich der Wicklungen in dem von den Wicklungen umgebenen Innenraum angeordnet ist, wobei die Innenraumschleife vorzugsweise als Kühlspirale ausgestaltet ist. Durch diese Innenraumschleife bzw. Kühlspirale kann sowohl die von den konzentrierten Wicklungen generierte Wärme, also beispielsweise die Kupferverluste, als auch die durch Eisenverluste in den Spulenkernen oder dem Rückschluss erzeugte Wärme effizient abgeführt werden, weil sie dort bzw. in unmittelbarer Nähe zu dem Ort aufgenommen wird, wo sie erzeugt wird.

Dabei ist es bevorzugt, dass die erste Kühlleitung und die zweite Kühlleitung in Serie miteinander verbunden sind. Speziell bevorzugt sind die erste Kühlleitung und die zweite Kühlleitung in ihrer Gesamtheit einstückig ausgestaltet, wobei die hintereinander bzw. in Serie angeordneten Kühlleitungen durch Biegen eines Rohrs hergestellt werden. Beispielsweise wird ein ursprünglich gerades Stück Rohr durch Biegen derart geformt, dass die U-förmigen Kühlschleifen mit den dazwischen angeordneten Verbindungssegmenten und die in Serie angeordnete zweite Kühlleitung entstehen.

In einer bevorzugten Ausgestaltung ist bezüglich der axialen Richtung zwischen den Wicklungen und den Querschenkeln eine erste Platine mit elektronischen Komponenten angeordnet, wobei die Kühlvorrichtung eine erste Ringleitung oder eine zweite Ringleitung umfasst, die jeweils von dem Kühlfluid durchströmbar sind, wobei sich die erste Ringleitung und die zweite Ringleitung jeweils in der Umfangsrichtung radial innenliegend entlang der Längsschenkel der Spulenkerne erstrecken, wobei die erste Ringleitung bezüglich der axialen Richtung zwischen der ersten Platine und den Querschenkeln angeordnet ist, und wobei die zweite Ringleitung bezüglich der axialen Richtung zwischen der ersten Platine und den Wicklungen angeordnet ist. Bei dieser Ausgestaltung ist also benachbart (bezüglich der axialen Richtung) zu den Querschenkeln eine erste Platine, beispielsweise ein Elektronikprint bzw. ein PCB (Printed Circuit Board) angeordnet, der z. B. elektronische Komponenten für die Ansteuerung oder den Betrieb von Sensoren, beispielsweise Positionssensoren oder Magnetfeldsensoren, enthalten kann, oder Komponenten für die Auswertung der von Sensoren gelieferten Signale.

Die elektronischen Komponenten, die auf einem Elektronikprint angeordnet sind, sollten besonders gut gegen eine Überhitzung geschützt werden, weil sie üblicherweise sehr empfindlich sind und bei einem zu grossen Wärmeeintrag beschädigt werden können, ausfallen können oder ihre Lebensdauer reduziert wird. Deshalb ist es vorteilhaft, die erste oder die zweite Ringleitung unmittelbar benachbart zu der ersten Platine anzuordnen, damit dieser Bereich gut gekühlt und die erste Platine mit ihren elektronischen Komponenten vor einer Überhitzung geschützt wird. Insbesondere wenn der Drehantrieb als Zentrifugalpumpe ausgestaltet ist, befindet sich die erste Platine in unmittelbarer Nachbarschaft zu dem Pumpengehäuse, in welchem der Rotor angeordnet ist. Beim Fördern von sehr heissen Fluiden, die beispielsweise eine Temperatur von bis zu 200°C oder mehr haben, wird eine erhebliche Wärmemenge von dem zu fördernden Fluid in den Drehantrieb und damit auch in die erste Platine eingetragen. Daher ist es bei solchen Anwendungen besonders vorteilhaft, die erste oder die zweite Ringleitung vorzusehen, damit die Wärme unmittelbar aus dem Bereich abgeführt wird, in welchem die erste Platine angeordnet ist.

Insbesondere sind auch solche Ausgestaltungen möglich, bei denen die Kühlvorrichtung die erste Ringleitung und die zweite Ringleitung aufweist.

Vorzugsweise sind die erste Ringleitung und oder die zweite Ringleitung integraler Bestandteil der zweiten Kühlleitung, und in Serie mit der Innenraumschleife bzw. der Kühlspirale verbunden.

In einer bevorzugten Ausführungsform ist eine zweite Platine mit elektronischen Komponenten vorgesehen, die bezüglich der axialen Richtung benachbart zu den ersten Enden der Längsschenkel auf der den Querschenkeln abgewandten Seite angeordnet ist, wobei die Kühlvorrichtung eine dritte Ringleitung oder eine vierte Ringleitung umfasst, die jeweils von dem Kühlfluid durchströmbar sind, wobei sich die dritte Ringleitung und die vierte Ringleitung jeweils in der Umfangsrichtung erstrecken, wobei die dritte Ringleitung bezüglich der axialen Richtung zwischen der zweiten Platine und den ersten Enden der Längsschenkel angeordnet ist, und wobei die vierte Ringleitung derart angeordnet ist, dass sich die zweite Platine bezüglich der axialen Richtung zwischen den ersten Enden der Längsschenkel und der vierten Ringleitung befindet..

Wenn man das erste Ende der Schenkel als unten bezeichnet und das zweite Ende der Schenkel als oben, dann ist die zweite Platine unterhalb der Spulenkerne angeordnet. Die dritte Ringleitung ist zwischen der zweiten Platine und den ersten Enden der Längsschenkel angeordnet, wobei letztere üblicherweise über den Rückschluss miteinander verbunden sind. Die vierte Ringleitung ist unterhalb der zweiten Platine angeordnet. Somit kann die dritte Ringleitung und/oder die vierte Ringleitung die zweite Platine mit ihren elektronischen Komponenten effizient vor einem zu grossen Wärmeeintrag schützen und auch Wärme aus dem Rückschluss abführen. Auf der zweiten Platine ist beispielsweise die Leistungselektronik zur Versorgung und zur Ansteuerung der Wicklungen vorgesehen.

Es sind Ausgestaltungen möglich, bei denen sowohl die dritte Ringleitung als auch die vierte Ringleitung vorgesehen sind, oder Ausgestaltungen, bei denen die dritte Ringleitung, nicht aber die vierte Ringleitung vorgesehen ist, oder Ausgestaltungen, bei denen die vierte Ringleitung nicht aber die dritte Ringleitung vorgesehen ist.

Vorzugsweise sind die dritte Ringleitung und/oder die vierte Ringleitung integraler Bestandteil der ersten Kühlleitung, und in Serie mit der mindestens einen Kühlschleife verbunden. Somit bedarf es nur zweier Kühlanschlüsse für das Kühlfluid, nämlich eines ersten Kühlanschlusses, durch welchen das Kühlfluid in die erste Kühlleitung und in die in Serie angeordnete zweite Kühlleitung eingebracht wird, und eines zweiten Kühlanschlusses, durch welchen das Kühlfluid nach Durchströmen der ersten und der zweiten Kühlleitung abgeführt wird.

Gemäss einer weiteren bevorzugten Ausführungsform sind an jedem Längsschenkel zwei konzentrierte Wicklungen vorgesehen, von denen jede den jeweiligen Längsschenkel umgibt, und die bezüglich der axialen Richtung benachbart zueinander angeordnet sind.

Eine weitere bevorzugte Ausgestaltung besteht darin, dass eine Mehrzahl von konzentrierten Antriebsspulen vorgesehen ist, von denen jede jeweils um die Längsschenkel zweier benachbarter Spulenkerne herum angeordnet ist, sodass die beiden Längsschenkel bezüglich der radialen Richtung innerhalb der Antriebsspule angeordnet sind. Diese Antriebsspulen sind somit auch als konzentrierte Wicklungen ausgestaltet, wobei jedoch jede Antriebsspule jeweils um zwei benachbarte Längsschenkel herum gewickelt ist, sodass die jeweilige Antriebsspule den Zwischenraum zwischen den beiden benachbarten Längsschenkeln umschliesst.

Bei dieser Ausgestaltung ist es bevorzugt, dass mindestens einer der Kühlleitungsabschnitte oder eine der Kühlschleifen bezüglich der radialen Richtung innerhalb einer der Antriebsspulen angeordnet ist. Die Antriebsspule umgibt also diesen Kühlleitungsabschnitt oder diese Kühlschleife, wodurch der in der Antriebsspule angeordnete Kühlleitungsabschnitt bzw. die in der Antriebsspule angeordnete Kühlschleife besonders gut die von der Antriebswicklung generierte Wärme abführen können.

Durch die Erfindung wird ferner eine Zentrifugalpumpe zum Fördern eines Fluids vorgeschlagen, welche einen elektromagnetischen Drehantrieb umfasst, der erfindungsgemäss ausgestaltet ist, wobei der Rotor des elektromagnetischen Drehantriebs als Rotor der Zentrifugalpumpe ausgestaltet ist.

Vorzugsweise umfasst die Zentrifugalpumpe ein Pumpengehäuse, das einen Pumpeneinlass und einen Pumpenauslass für das zu fördernde Fluid umfasst, wobei der Rotor im Pumpengehäuse angeordnet ist, und eine Mehrzahl von Flügeln zum Fördern des Fluids umfasst. Das Pumpengehäuse ist derart ausgestaltet, dass es so in den Stator einsetzbar ist, dass der magnetisch wirksame Kern des Rotors von den Querschenkeln umgeben wird.

Weitere vorteilhafte Massnahmen und Ausgestaltungen der Erfindung ergeben sich aus den abhängigen Ansprüchen.

Im Folgenden wird die Erfindung anhand von Ausführungsbeispielen und anhand der Zeichnung näher erläutert. In der Zeichnung zeigen:
- Fig. 1:: eine perspektivische Darstellung eines erstes Ausführungsbeispiels eines erfindungsgemässen elektromagnetischen Drehantriebs,
- Fig. 2:: eine perspektivische Darstellung der Kühlvorrichtung des ersten Ausführungsbeispiels,
- Fig. 3: eine perspektivische Darstellung eines zweiten Ausführungsbeispiels eines erfindungsgemässen elektromagnetischen Drehantriebs, teilweise im Schnitt,
- Fig. 4:: eine perspektivische Darstellung der Kühlvorrichtung des zweiten Ausführungsbeispiels,
- Fig. 5:: eine perspektivische Darstellung eines dritten Ausführungsbeispiels eines erfindungsgemässen elektromagnetischen Drehantriebs, teilweise im Schnitt,
- Fig. 6:: eine perspektivische Darstellung der Kühlvorrichtung des dritten Ausführungsbeispiels,
- Fig. 7:: eine perspektivische Darstellung einer Variante des dritten Ausführungsbeispiels, teilweise im Schnitt,
- Fig. 8:: eine perspektivische Darstellung eines vierten Ausführungsbeispiels eines erfindungsgemässen elektromagnetischen Drehantriebs,
- Fig. 9:: eine perspektivische Darstellung der Kühlvorrichtung des vierten Ausführungsbeispiels,
- Fig. 10:: eine perspektivische Darstellung eines fünften Ausführungsbeispiels eines erfindungsgemässen elektromagnetischen Drehantriebs, teilweise im Schnitt,
- Fig. 11:: eine perspektivische Darstellung der Kühlvorrichtung des fünften Ausführungsbeispiels in einer Explosionsdarstellung,
- Fig. 12:: eine perspektivische Darstellung eines Ausführungsbeispiels einer erfindungsgemässen Zentrifugalpumpe, und
- Fig. 13:: eine schematische Schnittdarstellung des Ausführungsbeispiels aus Fig. 12 in einem Schnitt in axialer Richtung.

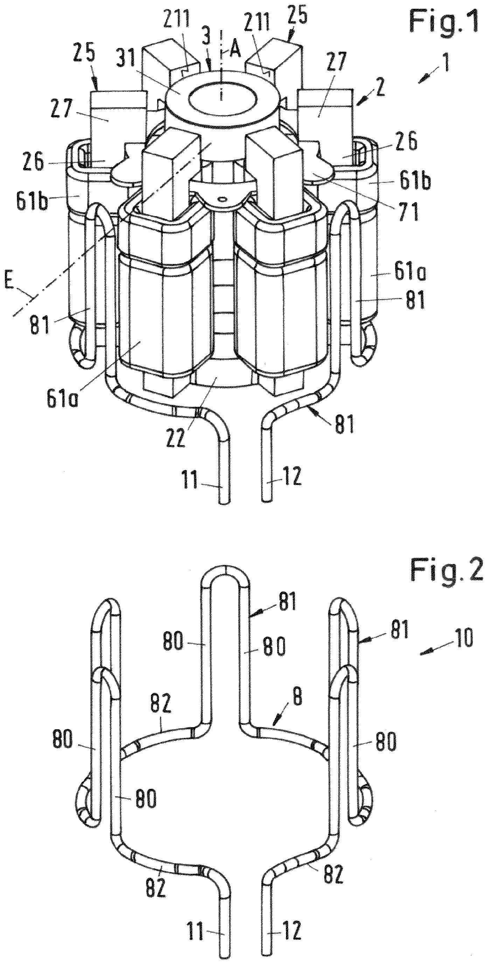

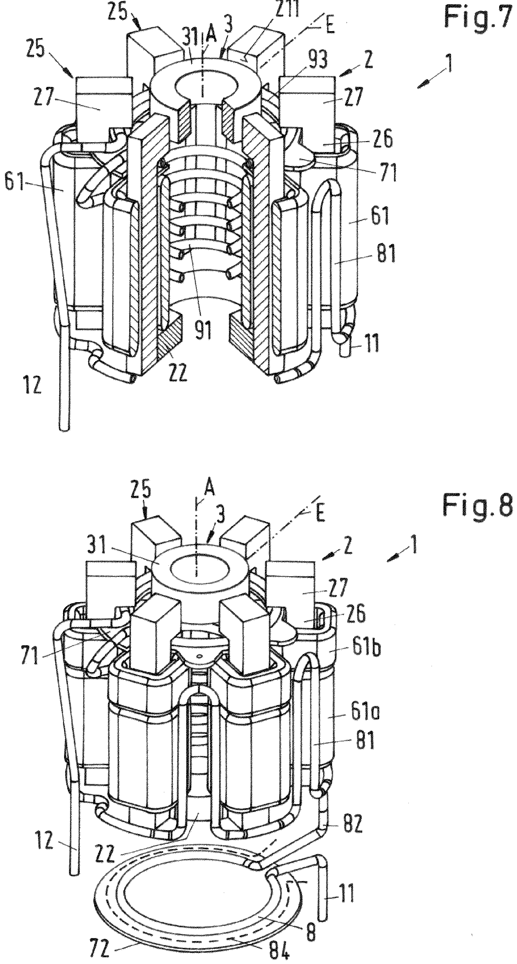

Fig. 1 zeigt eine perspektivische Darstellung eines ersten Ausführungsbeispiels eines erfindungsgemässen elektromagnetischen Drehantriebs, der gesamthaft mit dem Bezugszeichen 1 bezeichnet ist. Der elektromagnetische Drehantrieb 1 ist als Tempelmotor ausgestaltet und umfasst einen Stator 2, welcher eine Mehrzahl von Spulenkernen 25 - hier sechs Spulenkerne 25 - aufweist, von denen jeder einen Längsschenkel 26 umfasst, welcher sich in axialer Richtung A erstreckt, sowie einen senkrecht zum Längsschenkel 26 angeordneten Querschenkel 27, welcher sich in einer radialen Richtung erstreckt, und durch eine Stirnfläche 211 begrenzt wird. Die Spulenkerne 25 sind äquidistant auf einer Kreislinie angeordnet, sodass die Stirnflächen 211 einen Rotor 3 des elektromagnetischen Drehantriebs 1 umgeben. An jedem Längsschenkel 26 sind jeweils zwei konzentrierte Wicklungen 61a, 61b vorgesehen sind, von denen jede den jeweiligen Längsschenkel 26 umgibt, und die bezüglich der axialen Richtung A benachbart zueinander angeordnet sind. Bei anderen Ausführungsformen (siehe z. B. Fig. 7) ist an jedem Längsschenkel 26 genau eine konzentrierte Wicklung 61 angeordnet, welche den jeweiligen Längsschenkel 26 umgibt.

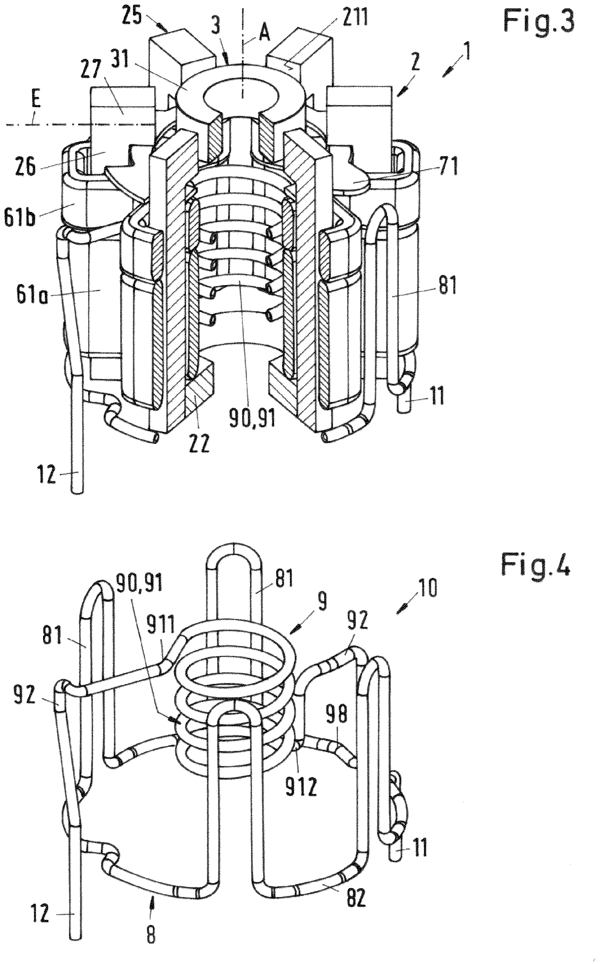

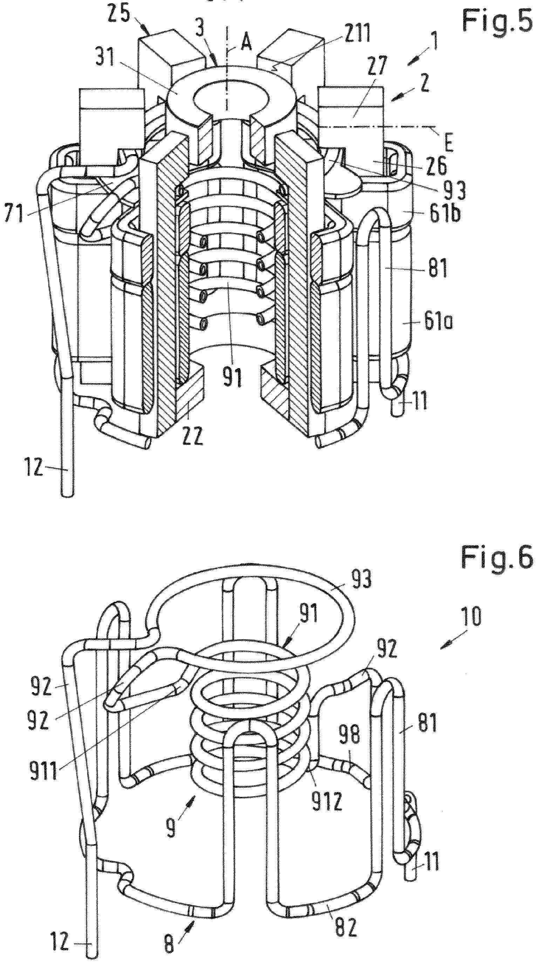

Die Längsschenkel 26 sind jeweils stabförmig oder im Wesentlich stabförmig ausgestaltet. Insbesondere können die Längsschenkel 26 auch so ausgestaltet sein, wie es beispielsweise in der Fig. 3 oder in der Fig. 5 der EP 4 084 304 A1 dargestellt ist.

Der Rotor 3 ist bezüglich des Stators 2 berührungslos magnetisch gelagert. Ferner ist der Rotor 3 mittels des Stators 2 berührungslos magnetisch zur Rotation um eine Solldrehachse antreibbar. Die Solldrehachse bezeichnet dabei diejenige Achse, um welche sich der Rotor 3 im Betriebszustand dreht, wenn sich der Rotor 3 bezüglich des Stators 2 in einer zentrierten und unverkippten Lage befindet, so wie dies in Fig. 1 dargestellt ist. Diese Solldrehachse definiert die axiale Richtung A. Üblicherweise stimmt die die axiale Richtung A festlegende Solldrehachse mit der Mittelachse des Stators 2 überein.

Im Folgenden wird mit einer radialen Richtung eine Richtung bezeichnet, welche senkrecht auf der axialen Richtung A steht.

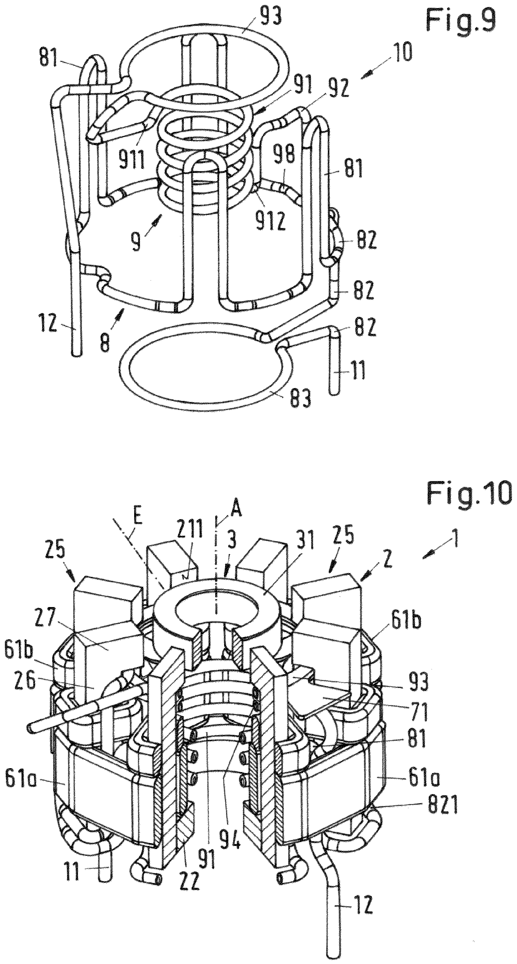

Es versteht sich, dass die Anzahl von sechs Spulenkernen 25 nur beispielhaft zu verstehen ist. Es sind natürlich auch solche Ausgestaltungen möglich, bei denen der Stator 2 weniger als sechs, z. B. fünf Spulenkerne 25 aufweist, oder solche Ausgestaltungen, bei denen der Stator 2 mehr als sechs, z. B. sieben oder acht Spulenkerne 25 aufweist (siehe z. B. Fig. 10).

Der Rotor 3 umfasst einen magnetisch wirksamen Kern 31, welcher ring- oder scheibenförmig ausgestaltet ist. Der magnetisch wirksame Kern 31 ist gemäss der Darstellung in Fig. 1 als Ring ausgestaltet und definiert eine magnetische Mittelebene. Alternativ kann der magnetisch wirksame Kern 31 auch als Scheibe ausgestaltet sein. In der Regel ist bei einem scheibenförmigen oder ringförmigen magnetisch wirksamen Kern 31 die magnetische Mittelebene die geometrische Mittelebene des magnetisch wirksamen Kerns 31 des Rotors 3, die senkrecht zur axialen Richtung A liegt. Im Betriebszustand ist der magnetisch wirksame Kern 31 in einer radialen Ebene gelagert, welche senkrecht auf der axialen Richtung A steht. Die radiale Ebene ist in Fig. 1 durch die Linie E angedeutet, die senkrecht auf der axialen Richtung A steht. Die radiale Ebene E ist also diejenige Ebene, welche senkrecht auf der axialen Richtung A steht und die Linie E enthält.

Die radiale Ebene E ist diejenige Ebene, in welcher der magnetisch wirksame Kern 31 des Rotors 3 im Betriebszustand zwischen den Stirnflächen 211 im Stator 2 aktiv magnetisch gelagert ist. Wenn der Rotor 3 nicht verkippt und in axialer Richtung A nicht ausgelenkt ist, liegt die magnetische Mittelebene in der radialen Ebene E. Die radiale Ebene E definiert die x-y-Ebene eines kartesischen Koordinatensystems, dessen z-Achse in axialer Richtung A verläuft.

Mit der radialen Position des magnetisch wirksamen Kerns 31 bzw. des Rotors 3 wird die Lage des Rotors 3 in der radialen Ebene E bezeichnet.

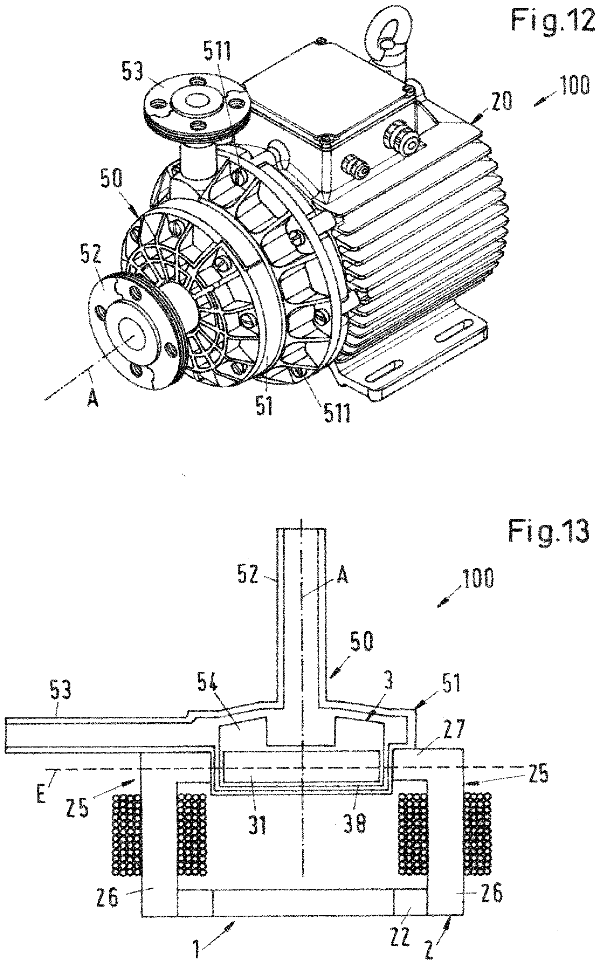

Da es für das Verständnis der Erfindung ausreichend ist, ist in der Zeichnung in den Fig. 1, 3, 5, 7, 8 und 10 von dem Rotor 3 jeweils nur der magnetisch wirksame Kern 31 dargestellt. Es versteht sich, dass der Rotor 3 natürlich auch noch weitere Komponenten umfassen kann wie beispielsweise Ummantelungen oder Kapselungen, die vorzugsweise aus einem Kunststoff hergestellt sind, oder aus einem Metall oder aus einer Metalllegierung oder aus einer Keramik bzw. einem keramischen Werkstoff. Ferner kann der Rotor 3 auch Flügel zum Mischen, Rühren oder Pumpen von Fluiden (siehe z. B. Fig. 13) oder sonstige Komponenten umfassen.

Der Stator 2 ist üblicherweise in einem Statorgehäuse 20 angeordnet (siehe Fig. 12), welches den Stator 2 vollständig umschliesst, sodass der gesamte Stator 2 im Inneren des Statorgehäuses 20 angeordnet ist. Zum besseren Verständnis ist in den Fig. 1, 3, 5, 7, 8 und 10 das Statorgehäuse 20 nicht dargestellt, sodass die Komponenten des im Statorgehäuses 20 angeordneten Stators 2 erkennbar sind.

Besonders bevorzugt ist das Statorgehäuse 20 als ein hermetisch dichtes Statorgehäuse 20 ausgestaltet, welches den Stator 2 vollständig umkapselt. Das Statorgehäuse 20 ist vorzugsweise mit einer thermisch leitenden Vergussmasse, beispielsweise mit einem Epoxidharz oder mit einem Polyurethan, aufgefüllt, sodass der Stator 2 und gegebenenfalls weitere Komponenten, die im Inneren des Statorgehäuses 20 angeordnet sind, mit der Vergussmasse umgeben sind. Dadurch wird der allgemeine thermische Widerstand reduziert und Vibrationen werden gedämpft.

Der Rotor 3 und insbesondere der magnetisch wirksame Kern 31 des Rotors 3 sind von den radial aussenliegend angeordneten Querschenkeln 27 der Spulenkerne 25 des Stators 2 umgeben. Die Querschenkel 27 bilden somit eine Mehrzahl von ausgeprägten Statorpolenhier sechs Statorpole. Die Längsschenkel 26 der Spulenkerne 25 erstrecken sich jeweils von einem ersten Ende, welches das darstellungsgemäss untere Ende ist, in axialer Richtung A bis zu einem zweiten Ende, welches das darstellungsgemäss obere Ende ist. Die Querschenkel 27 sind an den oberen Enden der Längsschenkel 26 und in der radialen Ebene E angeordnet. Jeder Querschenkel 27 erstreckt sich in der radialen Richtung auf den Rotor 3 zu.

Wenn sich der magnetisch wirksame Kern 31 des Rotors 3 während des Betriebs in seiner Soll-Position befindet, so ist der magnetisch wirksame Kern 31 zwischen den Stirnflächen 211 der Querschenkel 27 zentriert, sodass die in der radialen Ebene E angeordneten Querschenkel 27 auch in der magnetischen Mittelebene liegen. Die konzentrierten Wicklungen 61a, 61b sind darstellungsgemäss unterhalb der radialen Ebene E angeordnet und so ausgerichtet, dass ihre Spulenachsen in axialer Richtung A verlaufen.

Alle ersten Enden der Längsschenkel 26 - also die darstellungsgemäss unteren Enden - sind durch einen Rückschluss 22 miteinander verbunden. Der Rückschluss 22 ist vorzugsweise ringförmig ausgestaltet. Dabei sind solche Ausgestaltungen möglich (siehe Fig. 1), bei welchen sich der Rückschluss 22 radial innenliegend entlang aller ersten Enden der Längsschenkel 26 erstreckt. Es ist aber auch möglich, dass der Rückschluss 22 entlang seines Umfangs mehrere Ausnehmungen aufweist, von denen jede eines der ersten Enden aufnimmt. In anderen Ausführungsformen kann der Rückschluss auch eine Mehrzahl von Ringsegmenten umfassen, von denen jedes jeweils zwischen zwei in Umfangsrichtung benachbarten Spulenkernen 25 im Bereich der ersten Enden angeordnet ist.

Um die für den magnetischen Antrieb und die magnetische Lagerung des Rotors 3 notwendigen elektromagnetischen Drehfelder zu erzeugen, tragen die Längsschenkel 26 der Spulenkerne 25 die als konzentrierte Wicklungen 61a, 61b ausgestalteten Wicklungen, wobei bei dem ersten Ausführungsbeispiel um jeden Längsschenkel 26 herum jeweils genau zwei konzentrierte Wicklung 61a, 61b angeordnet sind, die bezüglich der axialen Richtung A benachbart sind. Mit diesen konzentrierten Wicklungen 61a, 61b werden im Betriebszustand diejenigen elektromagnetischen Drehfelder erzeugt, mit welchen ein Drehmoment auf den Rotor 3 bewirkt wird, und mit welchen eine beliebig einstellbare Querkraft in radialer Richtung auf den Rotor 3 ausübbar ist, sodass die radiale Position des Rotors 3, also seine Position in der zur axialen Richtung A senkrechten radialen Ebene E, aktiv steuerbar bzw. regelbar ist.

Mit dem "magnetisch wirksamen Kern 31" des Rotors 3 ist derjenige Bereich des Rotors 3 gemeint, welcher für die Drehmomentbildung sowie für die Erzeugung der magnetischen Lagerkräfte magnetisch mit dem Stator 2 zusammenwirkt.

Wie bereits erwähnt, ist der magnetisch wirksame Kern 31 bei diesem Ausführungsbeispiel ringförmig ausgestaltet. Ferner ist der magnetisch wirksame Kern 31 permanentmagnetisch ausgestaltet. Dazu kann der magnetisch wirksame Kern 31 mindestens einen Permanentmagneten, aber auch mehrere Permanentmagnete umfassen oder - wie im hier beschriebenen Ausführungsbeispiel - vollständig aus einem permanentmagnetischen Material bestehen, sodass der magnetisch wirksame Kern 31 der Permanentmagnet ist. Der magnetisch wirksame Kern 31 ist beispielsweise in radialer Richtung magnetisiert.

Als Permanentmagnete bezeichnet man üblicherweise solche ferromagnetischen oder ferrimagnetischen Werkstoffe, die hartmagnetisch sind, also eine hohe Koerzitivfeldstärke aufweisen. Die Koerzitivfeldstärke ist diejenige magnetische Feldstärke, die man benötigt, um einen Stoff zu entmagnetisieren. Im Rahmen dieser Anmeldung wird unter einem Permanentmagneten ein Werkstoff bzw. ein Material verstanden, der eine Koerzitivfeldstärke, genauer gesagt eine Koerzitivfeldstärke der magnetischen Polarisation aufweist, die mehr als 10'000 A/m beträgt.

Es sind auch solche Ausgestaltungen möglich, bei denen der magnetisch wirksame Kern 31 permanentmagnetfrei, also ohne Permanentmagnete ausgestaltet ist. Der Rotor 3 ist dann z.B. als Reluktanzläufer ausgestaltet. Der magnetisch wirksame Kern 31 des Rotors 3 besteht dann beispielsweise aus einem weichmagnetischen Material. Geeignete weichmagnetische Materialien für den magnetisch wirksamen Kern 31 sind beispielsweise ferromagnetische oder ferrimagnetische Materialien, also insbesondere Eisen, Nickel-Eisen, Kobalt-Eisen, Silizium-Eisen, Mu-Metall.

Ferner sind Ausgestaltungen möglich, bei denen der magnetisch wirksame Kern 31 des Rotors 3 sowohl ferromagnetische Materialien als auch permanentmagnetische Materialien umfasst. Beispielsweise können Permanentmagnete in einen ferromagnetischen Grundkörper eingelegt bzw. eingesetzt werden. Solche Ausgestaltungen sind z.B. vorteilhaft, wenn man bei grossen Rotoren die Kosten durch Einsparen von permanentmagnetischem Material reduzieren will.

Auch sind Ausgestaltungen möglich, bei denen der Rotor nach dem Prinzip eines Käfigläufers ausgestaltet ist.

Sowohl der ringförmige Rückschluss 22 als auch die Spulenkerne 25 des Stators 2 sind jeweils aus einem weichmagnetischen Material gefertigt, weil sie als Flussleitelemente zur Führung des magnetischen Flusses dienen.

Geeignete weichmagnetische Materialien für die Spulenkerne 25 und den Rückschluss 22 sind beispielsweise ferromagnetische oder ferrimagnetische Materialien, also insbesondere Eisen, Nickel-Eisen, Kobalt-Eisen Silizium-Eisen oder Mu-Metall. Hierbei ist für den Stator 2 eine Ausgestaltung als Statorblechpaket bevorzugt, bei welcher die Spulenkerne 25 und der Rückschluss 22, geblecht ausgestaltet sind, das heisst sie bestehen aus mehreren dünnen Blechelementen, die gestapelt sind.

Ferner ist es möglich, dass die Spulenkerne 25 und der Rückschluss 22 aus gepressten und anschliessend gesinterten Körnern der genannten Materialien bestehen. Die metallischen Körner sind dabei vorzugsweise in eine Kunststoffmatrix eingebettet, sodass sie zumindest teilweise gegeneinander isoliert sind, wodurch sich Wirbelstromverluste minimieren lassen. Es sind also für den Stator auch weichmagnetische Verbundwerkstoffe geeignet, welche aus elektrisch isolierten und zusammengepressten Metallpartikeln bestehen. Insbesondere können diese weichmagnetischen Verbundwerkstoffe, die auch als SMC (Soft Magnetic Composites) bezeichnet werden, aus Eisenpulverpartikeln bestehen, die mit einer elektrisch isolierenden Schicht beschichtet sind. Diese SMC werden dann mittels pulvermetallurgischer Verfahren zu der gewünschten Ausgestaltung geformt.

Während des Betriebs des elektromagnetischen Drehantriebs 1 wirkt der magnetisch wirksame Kern 31 des Rotors 3 mit dem Stator 2 nach dem eingangs beschriebenen Prinzip des lagerlosen Motors zusammen, bei welchem der Rotor 3 berührungslos magnetisch antreibbar und berührungslos magnetisch bezüglich des Stators 2 lagerbar ist. Dazu ist der Stator 2 als Lager- und Antriebsstator ausgestaltet, mit welchem der Rotor 3 im Betriebszustand berührungslos magnetisch um die Solldrehachse antreibbar - also in Rotation versetzbar- und bezüglich des Stators 2 berührungslos magnetisch lagerbar ist. Dabei sind drei Freiheitsgrade des Rotors 3, nämlich seine Position in der radialen Ebene E und seine Rotation, aktiv regelbar. Bezüglich seiner axialen Auslenkung aus der radialen Ebene E in axialer Richtung A ist der magnetisch wirksame Kern 31 des Rotors 3 passiv magnetisch, das heisst nicht ansteuerbar, durch Reluktanzkräfte stabilisiert. Auch bezüglich der verbleibenden zwei Freiheitsgrade, nämlich Verkippungen bezüglich der zur Solldrehachse senkrechten radialen Ebene E ist der magnetisch wirksame Kern 31 des Rotors 3 ebenfalls passiv magnetisch stabilisiert. Der Rotor 3 ist also durch das Zusammenwirken des magnetisch wirksamen Kerns 31 mit den Spulenkernen 25 in axialer Richtung A sowie gegen Verkippungen (insgesamt drei Freiheitsgrade) passiv magnetisch gelagert oder passiv magnetisch stabilisiert und in der radialen Ebene (zwei Freiheitsgrade) aktiv magnetisch gelagert.

Wie dies allgemein üblich ist, bezeichnet auch im Rahmen dieser Anmeldung eine aktive magnetische Lagerung eine solche, die aktiv steuer- bzw. regelbar ist, beispielsweise über die mit den konzentrierten Wicklungen 61a, 61b generierten elektromagnetischen Drehfelder. Eine passive magnetische Lagerung oder eine passive magnetische Stabilisierung bezeichnet eine solche, die nicht ansteuerbar bzw. regelbar ist. Die passive magnetische Lagerung oder Stabilisierung basiert beispielsweise auf Reluktanzkräften, welche den Rotor 3 bei einer Auslenkung aus seiner Soll-Position, also z. B. bei einer Verschiebung oder Auslenkung in axialer Richtung A oder bei einer Verkippung, wieder in seine Soll-Position bringen.

Mit einer Radiallagerung oder einer radialen Lagerung wird eine Lagerung des Rotors 3 bezeichnet, mit welcher die radiale Position des Rotors 3 stabilisiert werden kann, also eine Lagerung, welche den Rotor 3 in der radialen Ebene E und damit bezüglich seiner radialen Position lagert.

Mit einer Axiallagerung oder einer axialen Lagerung bzw. mit einer Axialstabilisierung oder einer axialen Stabilisierung wird eine Lagerung bzw. eine Stabilisierung des Rotors 3 bezeichnet, mit welcher zum einen die Position des Rotors 3 bezüglich der axialen Richtung A stabilisiert wird, und mit welcher zum anderen der Rotor 3 gegen Verkippungen stabilisiert ist. Solche Verkippungen stellen zwei Freiheitsgrade dar und bezeichnen Auslenkungen, bei denen die momentane Drehachse des Rotors 3 nicht mehr genau in die axiale Richtung A zeigt, sondern einen von Null verschiedenen Winkel mit der Solldrehachse einschliesst. Bei einer Verkippung liegt also die magnetische Mittelebene C nicht mehr in oder parallel zur radialen Ebene E, sondern die magnetische Mittelebene C schliesst einen von Null verschiedenen Winkel mit der radialen Ebene E ein.

Beim lagerlosen Motor werden im Unterschied zu klassischen Magnetlagern die magnetische Lagerung und der Antrieb des Motors über elektromagnetische Drehfelder realisiert. Typischerweise wird in dem lagerlosen Motor die magnetische Antriebs- und Lagerfunktion durch die Überlagerung zweier magnetischer Drehfelder generiert, die üblicherweise als Antriebs- und Steuerfeld bezeichnet werden. Diese beiden mit den Wicklungen des Stators 2 erzeugten Drehfelder haben in der Regel eine Polpaarzahl, die sich um eins unterscheidet.

Hat also beispielsweise das Antriebsfeld die Polpaarzahl p, so hat das Steuerfeld die Polpaarzahl p+1 oder p-1. Dabei werden mit dem Antriebsfeld auf den magnetisch wirksamen Kern 31 in der radialen Ebene wirkende Tangentialkräfte erzeugt, die ein Drehmoment bewirken, was die Rotation um die axiale Richtung A bewirkt. Durch die Überlagerung des Antriebsfelds und des Steuerfelds lässt sich zusätzlich eine beliebig einstellbare Querkraft auf den magnetisch wirksame Kern 31 in der radialen Ebene erzeugen, mit welcher die Position des magnetisch wirksamen Kerns 31 in der radialen Ebene regelbar ist. Es ist also nicht möglich, den elektromagnetischen Fluss, der von den konzentrierten Wicklungen 61a, 61b generiert wird, aufzuteilen in einen (elektro-) magnetischen Fluss, der nur für den Antrieb der Rotation sorgt und einen (elektro-) magnetischen Fluss, der nur die magnetische Lagerung realisiert.

Zur Generierung des Antriebs- und des Steuerfelds ist es einerseits möglich - wie in Fig 1 gezeigt-, zwei unterschiedliche Wicklungssysteme zu verwenden, nämlich eines zur Erzeugung des Antriebsfelds und eines zur Erzeugung des Steuerfelds. Die Spulen zur Erzeugung des Antriebsfelds werden dann üblicherweise als Antriebsspulen 61a bezeichnet und die Spulen zur Erzeugung des Steuerfelds als Steuerspulen 61b.

So kann beispielsweise eine der konzentrierten Wicklungen als Antriebsspule 61a eingesetzt werden, während die andere, zu ihr benachbarte konzentrierte Wicklung als Steuerspule 61b eingesetzt wird. Der Strom, der in diese Spulen eingeprägt wird, wird dann als Antriebsstrom bzw. als Steuerstrom bezeichnet. Mit der Gesamtheit aller Antriebsspulen 61a wird dann das Antriebsfeld generiert, welches ein elektromagnetisches Drehfeld ist, während mit der Gesamtheit der Steuerspulen 61b das Steuerfeld generiert wird, welches ebenfalls ein elektromagnetisches Drehfeld ist, und welches dem Antriebsfeld überlagert wird. Durch die Überlagerung des Antriebsfelds und des Steuerfelds lassen sich dann die Tangentialkräfte auf den Rotor 3 generieren, welche das Drehmoment zum Antreiben der Rotation erzeugen, sowie eine beliebig einstellbare Querkraft in der radialen Ebene E, mit welcher die Position des magnetisch wirksamen Kerns 31 in der radialen Ebene aktiv regelbar ist.

Es sind aber auch solche Ausgestaltungen möglich (siehe z.B. Fig. 7), bei denen die Antriebs- und Lagerfunktion mit nur einem einzigen Wicklungssystem generiert wird, sodass es also keine Unterscheidung zwischen Antriebs- und Steuerspulen gibt. Dies kann so realisiert werden, dass die von einer Kontrolleinrichtung jeweils ermittelten Werte für den Antriebs- und den Steuerstrom rechnerisch -also z. B. mit Hilfe von Software - addiert bzw. überlagert werden und der sich daraus ergebende Gesamtstrom in die jeweilige konzentrierte Wicklung 61 (siehe Fig. 7) eingeprägt wird. In diesem Fall ist es natürlich nicht mehr möglich, zwischen Steuer- und Antriebsspulen zu unterscheiden, sondern an jedem Längsschenkel 26 ist jeweils genau eine konzentrierte Wicklung 61 (Fig. 7) vorgesehen. Es gibt dann nur ein Wicklungssystem, in dessen konzentrierten Wicklungen 61 die rechnerisch ermittelte Summe aus dem Antriebs- und dem Steuerstrom eingeprägt wird.

Je nach Anwendungsfall kann die Ausgestaltung mit separaten Antriebsspulen 61a und separaten Steuerspulen 61b die Ansteuerung bzw. die Regelung des elektromagnetischen Drehantriebs 1 vereinfachen.

Bei dem ersten Ausführungsbeispiel ist ferner eine erste Platine 71 mit nicht näher dargestellten elektronischen Komponenten vorgesehen. Die erste Platine 71 ist vorzugsweise als Elektronikprint bzw. PCB (printed circuit board) ausgestaltet. Auf der ersten Platine 71 können beispielsweise solche Komponenten vorgesehen sein, die für die Ansteuerung von Sensoren (nicht dargestellt) und/oder für die Auswertung der von Sensoren ermittelten Messsignale verwendet werden. Solche Sensoren umfassen beispielsweise Positionssensoren zur Bestimmung der aktuellen Position des Rotors 2.

Die erste Platine 71 ist im Wesentlichen ringförmig ausgestaltet und parallel zur radialen Ebene E angeordnet. Vorzugsweise ist die ringförmige erste Platine 71 radial innenliegend bezüglich der Spulenkerne 25 angeordnet, wobei sich die erste Platine 71 - wie in Fig. 1 zu erkennen ist - auch in die Zwischenräume zwischen den Längsschenkeln 26 benachbarter Spulenkerne 25 hinein erstrecken kann.

Bezüglich der axialen Richtung A ist die erste Platine 71 zwischen den Wicklungen 61b und den Querschenkeln 27 der Spulenkerne 25 angeordnet. Gemäss der Darstellung in Fig. 1 ist die erste Platine 71 also unterhalb und benachbart zu den Querschenkeln 27 angeordnet, welche um den magnetisch wirksamen Kern 31 des Rotors 3 herum angeordnet sind.

Erfindungsgemäss umfasst der elektromagnetische Drehantrieb 1 eine Kühlvorrichtung 10 (Fig. 2), die zumindest eine erste Kühlleitung 8 umfasst, welche von einem Kühlfluid durchströmbar ist. Die erste Kühlleitung 8 weist mindestens einen Kühlleitungsabschnitt 80 auf, welcher bezüglich der Umfangsrichtung in dem Zwischenraum zwischen den Längsschenkeln 26 zweier benachbarter Spulenkerne 25 angeordnet ist, und welcher sich in der axialen Richtung A erstreckt. Im Wesentlich erstreckt sich jeder Kühlleitungsabschnitt 80 entlang einer der Längsschenkel 26 und ist benachbart zu diesem angeordnet.

Bei der in den Fig. 1 und Fig. 2 dargestellten Anordnung sind jeweils zwei, im Wesentlichen parallel zueinander verlaufende Kühlleitungsabschnitte 80 durch eine gekrümmte Verbindung zu einer Kühlschleife 81 verbunden, sodass das Kühlfluid in einem der beiden Kühlleitungsabschnitte 80 darstellungsgemäss nach oben strömt, und in dem anderen der beiden Kühlleitungsabschnitte 80 darstellungsgemäss nach unten strömt. Jede Kühlschleife 81, welche jeweils zwei der Kühlleitungsabschnitte 80 umfasst, ist bezüglich der Umfangsrichtung in genau einem der Zwischenräume zwischen den Längsschenkeln 26 zweier benachbarter Spulenkerne 25 angeordnet.

Bei anderen Ausgestaltungen ist es auch möglich, dass sich mindestens eine der Kühlschleifen 81 durch mehr als einen der Zwischenräume zwischen den Längsschenkeln 26 zweier benachbarter Spulenkerne 25 erstreckt. Beispielsweise kann einer der Kühlleitungsabschnitte 80 der Kühlschleife 81 in einem der Zwischenräume angeordnet sein, und der andere Kühlleitungsabschnitt 80 der zur selben Kühlschleife 81 gehört, ist in einem anderen Zwischenraum, beispielsweise einem benachbarten Zwischenraum angeordnet. Die gekrümmte Verbindung zwischen diesen beiden Kühlleitungsabschnitten 80 kann dann beispielsweise darstellungsgemäss oberhalb derjenigen Wicklung 61b angeordnet sein, welche diese beiden Zwischenräume voneinander trennt.

Durch die in Fig. 1 dargestellte Anordnung der Kühlschleife 81 zwischen benachbarten Längsschenkeln 26 ist es möglich, die Wärme in den Bereichen aufzunehmen und aus diesen abzuführen, wo wesentliche Wärmequellen sind. Im Betrieb des Drehantriebs wird nämlich durch die Eisenverluste Wärme in den Spulenkernen 25 generiert, und durch die Kupferverluste wird Wärme in den konzentrierten Wicklungen 61a, 61b generiert, welche um die Längsschenkel 26 herum angeordnet sind. Da die Kühlschleife 81 in dem Zwischenraum zwischen zwei benachbarten Längsschenkeln 25 und damit auch im Zwischenraum zwischen den konzentrierten Wicklungen 61a, 61b, welche diese benachbarten Längsschenkeln 26 tragen, angeordnet ist, wird die Wärme effizient aus dem Bereich abgeführt, wo sich im Betriebszustand eine Hauptwärmequelle befindet.

Das Kühlfluid ist vorzugsweise eine Flüssigkeit. Ein besonders bevorzugtes Kühlfluid ist demineralisiertes oder deionisiertes Wasser (DI Wasser). DI Wasser ist eine typische Kühlflüssigkeit, welche beispielsweise in den Produktionsanlagen der Halbleiterindustrie häufig verwendet wird.

Die Leitungen der Kühlvorrichtung 10, also beispielsweise die erste Kühlleitung 8 mit der Kühlschleife 81, bestehen vorzugsweise aus einem rostfreien Stahl oder aus einem Edelstahl. Je nach Anwendungsfall ist auch möglich, dass die Leitungen der Kühlvorrichtung 10 aus Kupfer oder Aluminium bestehen. Aber insbesondere bei der Verwendung von DI Wasser als Kühlfluid ist ein rostfreier Stahl oder ein Edelstahl für die Leitungen bevorzugt, weil dieser korrosionsbeständiger ist als beispielsweise Kupfer.

Zum besseren Verständnis zeigt Fig. 2 eine perspektivische Darstellung der Kühlvorrichtung 10 des ersten Ausführungsbeispiels. Die Kühlvorrichtung 10 umfasst die erste Kühlleitung 8 sowie zwei Kühlanschlüsse 11, 12, nämlich einen ersten Kühlanschluss 11 und einen zweiten Kühlanschluss 12, durch welche das Kühlfluid in die erste Kühlleitung 8 einbringbar bzw. aus der ersten Kühlleitung 8 abführbar ist. Die nicht im Detail dargestellten Kühlanschlüsse 11, 12 umfassen jeweils eine Durchführung durch das Statorgehäuse 20 (Fig. 12), sodass das Kühlfluid von ausserhalb des Statorgehäuses 20 mittels beispielsweise des ersten Kühlanschlusses 11 durch die Wandung des Statorgehäuses 20 in die erste Kühlleitung 8 eingespeist werden kann und mittels des zweiten Kühlanschlusses 12 wieder durch die Wandung des Statorgehäuses 20 hindurch abgeführt werden kann.

Bei dem ersten Ausführungsbeispiel umfasst die erste Kühlleitung 8 mehrere, hier nämlich fünf Kühlschleifen 81, von denen jede bezüglich der Umfangsrichtung jeweils in dem Zwischenraum zwischen zwei benachbarten Spulenkernen 25 angeordnet ist, wobei in jedem Zwischenraum höchstens eine Kühlschleife 81 vorgesehen ist. Alle Kühlschleifen 81 sind in Serie, also hintereinander angeordnet. Ferner umfasst die erste Kühlleitung 8 mehrere Verbindungssegmente 82 zum Verbinden benachbarter Kühlschleifen 81 und zur Verbindung mit den Kühlanschlüssen 11, 12.

Jede der Kühlschleifen 81 ist U-förmig ausgestaltet, wobei jeweils die geschlossene Seite des U benachbart zu den zweiten Enden der Längsschenkel 26 - also darstellungsgemäss (Fig. 1) oben - und die offene Seite des U benachbart zu den ersten Enden der Längsschenkel 26 - also darstellungsgemäss unten - angeordnet ist. Die beiden Schenkel des U erstrecken sich jeweils in axialer Richtung A und somit parallel zu den Längsschenkeln 26 der Spulenkerne 25. Der Abstand der beiden Schenkel jeder U-förmigen Kühlschleife 81 ist so bemessen, dass die Kühlschleife 81 in den Zwischenraum zwischen zwei in Umfangsrichtung benachbarten Wicklungen 61a bzw. 61b passt.

Zwischen zwei in Umfangsrichtung benachbarten Kühlschleifen 81 ist jeweils eines der Verbindungssegment 82 angeordnet, welches einen der Schenkel der U-förmigen Kühlschleife 81 an der offenen Seite des U mit dem benachbarten Schenkel der benachbarten U-förmigen Kühlschleife 81 verbindet, sodass alle Kühlschleifen 81 strömungstechnisch gesehen in Serie angeordnet sind.

Die Verbindungssegmente 82 zwischen benachbarten Kühlschleifen 81 bzw. zwischen den Kühlanschlüssen 11, 12 und den Kühlschleifen 81 erstrecken sich jeweils bogenförmig in Umfangsrichtung und sind radial aussenliegend und benachbart zu den ersten Enden der Längsschenkel 25 und dem Rückschluss 22 angeordnet. Bezüglich der axialen Richtung sind die Verbindungssegmente 82 auf der gleichen Höhe wie der Rückschluss 22 angeordnet, sodass die Verbindungssegmente 82 radial aussenliegend im Wesentlichen dem Verlauf des Rückschlusses 22 folgen. Hieraus resultiert auch eine besonders effiziente Wärmeabfuhr aus dem Bereich des Rückschlusses 22.

Insgesamt hat die erste Kühlleitung 8 mit den Kühlschleifen 81 und den Verbindungssegmenten 82 einen kreisförmigen Verlauf und erstreckt sich in Umfangsrichtung um den gesamten Stator 2 herum, was insbesondere in Fig. 2 gut zu erkennen ist. Die beiden Kühlanschlüsse 11, 12 sind benachbart zueinander angeordnet und erstrecken sich jeweils in axialer Richtung A, also parallel zueinander. Jeder Kühlanschluss 11, 12 erstreckt sich jeweils aus dem Innenraum des Statorgehäuses 20 (Fig. 12) durch die Wandung des Statorgehäuses 20 hindurch nach aussen. Die erste Kühlleitung 8 erstreckt sich von dem ersten Kühlanschluss 11 in Umfangsrichtung und radial aussenliegend benachbart zu dem Rückschluss 22 um den Stator 2 herum bis zu dem zweiten Kühlanschluss 12, wobei zwischen benachbarten Spulenkernen 25 die U-förmigen Kühlschleifen 81 vorgesehen sind, die sich jeweils parallel zu den Längsschenkeln 26 der Spulenkerne 25 darstellungsgemäss nach oben, beispielsweise bis an das obere axiale Ende der konzentrierten Wicklungen 61b oder bis kurz unterhalb der ersten Platine 71 erstrecken.

Wie bereits erwähnt, sind bei dem ersten Ausführungsbeispiel mit beispielhaftem Charakter genau sechs Spulenkerne 25 und genau fünf Kühlschleifen 81 vorgesehen. Bei sechs auf einer Kreislinie angeordneten Spulenkernen 25 gibt bezüglich der Umfangsrichtung insgesamt sechs Zwischenräume zwischen jeweils benachbarten Spulenkernen 25. In fünf dieser Zwischenräume ist jeweils eine der Kühlschleifen 81 vorgesehen, und die beiden parallel zueinander angeordneten Kühlanschlüsse 11, 12 sind bezüglich der axialen Richtung A darstellungsgemäss unterhalb desjenigen Zwischenraums angeordnet, in welchem keine Kühlschleife 81 vorgesehen ist.

Bei dem ersten Ausführungsbeispiel ist die Anzahl der Kühlschleifen 81 also um eins geringer als die Anzahl der Zwischenräume zwischen in Umfangsrichtung benachbarten Spulenkernen 25. Es sind aber auch solche Ausgestaltungen möglich, bei denen in jedem der Zwischenräume jeweils eine der Kühlschleifen 81 angeordnet ist. Auch sind Ausgestaltungen möglich, bei denen die Differenz aus der Anzahl der Zwischenräume zwischen in Umfangsrichtung benachbarten Spulenkernen 25 und der Anzahl der Kühlschleifen 81 grösser als eins ist. Beispielsweise sind Ausgestaltungen möglich, bei denen nur in jedem zweiten Zwischenraum eine Kühlschleife 81 vorgesehen ist, sodass in Umfangsrichtung gesehen zwischen zwei Zwischenräumen, in denen eine Kühlschleife 81 angeordnet ist, jeweils ein Zwischenraum ohne Kühlschleife liegt.

Die Anzahl der Kühlschleifen 81 und auch der Verlauf der ersten Kühlleitung 8 können je nach Anwendungsfall angepasst werden. Wesentliche Faktoren sind hierbei die Grösse des Drehantriebs 1, der im Stator 2 zur Verfügung stehende Raum und die benötigte Kühlleistung. Wenn beispielsweise der Drehantrieb 1 als Zentrifugalpumpe ausgestaltet ist und sehr heisse Fluide von beispielsweise mehr als 70°C gefördert werden müssen, ist zu berücksichtigen, dass das zu fördernde Fluid zusätzlich Wärme in den Stator 2 einträgt, sodass eine stärkere Wärmeabfuhr vorteilhaft ist.

Besonders bevorzugt ist die erste Kühlleitung 8 mit den Kühlschleifen 81 und den Verbindungssegmenten 82 einstückig ausgestaltet. Speziell bevorzugt wird die erste Kühlleitung 8 durch Biegen eines Rohres hergestellt. Dies kann beispielsweise derart erfolgen, dass ein ursprünglich gerades Rohr, z. B. ein Edelstahlrohr, durch Biegen derart geformt wird, dass die Kühlschleifen 81 und die Verbindungssegmente 82 entstehen. Es ist ersichtlich, dass die in Fig. 2 dargestellte Form der ersten Kühlleitung 8 durch Biegen eines ursprünglich geraden Rohres hergestellt werden kann.

Fig. 3 zeigt eine perspektivische Darstellung eines zweiten Ausführungsbeispiels eines erfindungsgemässen Drehantriebs 1. In Fig. 3 ist ein Segment aus dem Stator 2 herausgeschnitten, damit das Innere des von den Spulenkernen 25 umgebenen Raums erkennbar ist. Zum besseren Verständnis zeigt Fig. 4 in einer perspektivischen Darstellung die Kühlvorrichtung 10 des zweiten Ausführungsbeispiels.

Im Folgenden wird nur auf die Unterschiede zu dem ersten Ausführungsbeispiel eingegangen. Gleiche Teile oder von der Funktion her gleichwertige Teile des zweiten Ausführungsbeispiels sind mit den gleichen Bezugszeichen bezeichnet wie bei dem ersten Ausführungsbeispiel. Insbesondere haben die Bezugszeichen die gleiche Bedeutung wie sie bereits im Zusammenhang mit dem ersten Ausführungsbeispiel erläutert sind. Es versteht sich, dass alle vorangehenden Erläuterungen des ersten Ausführungsbeispiels in gleicher Weise oder in sinngemäss gleicher Weise auch für das zweite Ausführungsbeispiel gelten.

Bei dem zweiten Ausführungsbeispiel hat die erste Kühlleitung 8 vier Kühlschleifen 81, von denen jede in einem der Zwischenräume zwischen den Längsschenkeln 26 benachbarter Spulenkerne 25 angeordnet ist, wobei jede Kühlschleife 81 in einem anderen Zwischenraum angeordnet ist. Somit ist in vier der sechs Zwischenräume jeweils eine Kühlschleife 81 angeordnet, und die beiden verbleibenden Zwischenräume sind ohne Kühlschleife 81 ausgestaltet.

Die Kühlvorrichtung 10 umfasst ferner eine zweite Kühlleitung 9, die von dem Kühlfluid durchströmbar ist. Die zweite Kühlleitung 9 weist eine Innenraumschleife 90 auf, welche radial innenliegend bezüglich der Wicklungen 61a, 61b in dem von den Wicklungen 61a, 61b umgebenen Innenraum angeordnet ist. Die Innenraumschleife 90 ist vorzugsweise -wie in den Fig. 3 und Fig. 4 gezeigt- als Kühlspirale 91 ausgestaltet.

Die Kühlspirale 91 ist radial innenliegend bezüglich der konzentrierten Wicklungen 61a, 61b angeordnet ist, sodass die Wicklungen 61a, 61b um die Kühlspirale 91 herum angeordnet ist. Die Kühlspirale 91 ist in dem von den Spulenkernen 25 bzw. den Wicklungen 61a, 61b umgebenen Raum angeordnet und kann somit effizient die Wärme aus diesem Bereich abführen. Dies ist insbesondere auch die durch Kupferverluste in den Wicklungen 61a, 61b generierte Wärme.

Die Achse der Kühlspirale 91 verläuft in der axialen Richtung A. In an sich bekannter Art umfasst die Kühlspirale 91 mehrere Windungen, die in axialer Richtung A benachbart angeordnet sind, wobei alle Windungen den gleichen Durchmesser haben. Im Bereich der Kühlspirale 91 ist die zweite Kühlleitung 9 folglich schraubenlinienförmig ausgestaltet.

Die Kühlspirale 91 erstreckt sich von einem ersten Ende 911, welches das darstellungsgemäss obere Ende ist bis zu einem zweiten Ende 912, welches das darstellungsgemäss untere Ende ist. Der Aussendurchmesser der Kühlspirale 91 ist vorzugsweise so bemessen, dass er nur wenig kleiner ist als der Innendurchmesser des von den Wicklungen 61a, 61b umschlossenen Raums, sodass die Windungen der Kühlspirale 91 bezüglich der radialen Richtung unmittelbar benachbart zu den Wicklungen 61a, 61b angeordnet sind. Im Bereich der Kühlspirale 91 verläuft die zweite Kühlleitung 9 somit vom ersten Ende 911 schraubenförmig entlang der radial innenliegenden Oberflächen der Wicklungen 61a, 61b bis zum zweiten Ende 912.

Das erste Ende 911 der Kühlspirale 91 ist über ein Verbindungssegment 92 der zweiten Kühlleitung 9 mit dem zweiten Kühlanschluss 12 verbunden. Das zweite Ende 912 der Kühlspirale 91 ist über ein Verbindungssegment 92 der zweiten Kühlleitung 9 mit einem Verbindungselement 98 verbunden, welches die zweite Kühlleitung 9 mit der ersten Kühlleitung 8 verbindet, sodass die erste Kühlleitung 8 und die zweite Kühlleitung 9 in Serie miteinander verbunden sind. Die erste Kühlleitung 8 erstreckt sich von dem Verbindungselement 98 bis zu dem ersten Kühlanschluss 11.

Vorzugsweise sind die beiden Verbindungssegmente 92 der zweiten Kühlleitung 9, welche die Kühlspirale 91 mit dem zweiten Kühlanschluss 12 bzw. mit dem Verbindungselement 98 verbinden, so angeordnet, dass sie durch jeweils einen derjenigen Zwischenräume zwischen den Längsschenkeln 26 benachbarter Spulenkerne 25 verlaufen, in denen keine Kühlschleife 81 vorgesehen ist.

Auch die zweite Kühlleitung 9 besteht vorzugsweise aus einem rostfreien Stahl oder aus einem Edelstahl.

Das Kühlfluid wird durch einen der beiden Kühlanschlüsse 11, 12, beispielsweise durch den ersten Kühlanschluss 11 zugeführt, durchströmt die erste Kühlleitung 8 mit den Kühlschleifen 81, strömt dann durch das Verbindungselement 98 in die zweite Kühlleitung 9 und fliesst nach Durchströmen der zweite Kühlleitung 9 durch den zweiten Kühlanschluss 12 ab.

Besonders bevorzugt ist die zweite Kühlleitung 9 mit der Kühlspirale 91 und den Verbindungssegmenten 92 einstückig ausgestaltet. Speziell bevorzugt wird die zweite Kühlleitung 9 durch Biegen eines Rohres hergestellt. Dies kann beispielsweise derart erfolgen, dass ein ursprünglich gerades Rohr, z. B. ein Edelstahlrohr, durch Biegen derart geformt wird, dass die Kühlspirale 91 und die Verbindungssegmente 92 entstehen. Es ist ersichtlich, dass die in Fig. 4 dargestellte Form der zweiten Kühlleitung 9 durch Biegen eines ursprünglich geraden Rohres hergestellt werden kann.

Aus konstruktiven Gründen ist es bevorzugt, dass die erste Kühlleitung 8 und die zweite Kühlleitung 9 zunächst separat jeweils einstückig durch Biegen eines Rohres hergestellt und dann mittels des Verbindungselements 98 miteinander verbunden werden, sodass sie strömungstechnisch in Serie angeordnet sind.

Das Verbindungselement 98, welches die erste Kühlleitung 8 mit der zweiten Kühlleitung 9 verbindet, ist vorzugsweise als ein flexibles Verbindungselement 98 ausgestaltet, wodurch sich auch das Zusammenfügen der ersten Kühlleitung 8 und der zweiten Kühlleitung 9 vereinfacht. Das Verbindungselement 98 kann beispielsweise als eine Schlauch-Schellen Verbindung ausgestaltet sein, und ein Stück Schlauch geeigneter Länge sowie zwei Schellen umfassen. Mittels der Schellen wird das Stück Schlauch einerseits an der ersten Kühlleitung 8 fixiert und andererseits an der zweiten Kühlleitung 9. Als Schellen sind insbesondere 2-Ohr Schellen bzw. 2-Ohr Klemmen geeignet, die eine Rundumabdichtung an der Verbindung zwischen der ersten Kühlleitung 8 und der zweiten Kühlleitung 9 gewährleisten.

Eine weitere bevorzugte Massnahme besteht darin, dass die Kühlleitungen 8, 9 durch Isolationsfolien oder durch ein Isolierband, beispielsweise ein Isolations-Klebeband gegenüber den elektrischen oder elektronischen Komponenten geschützt werden, sodass es nicht zu unerwünschten elektrischen Strömen oder Aufladungen oder elektrischen Überschlägen in die Kühlvorrichtung 10 kommt.

Um die Betriebssicherheit noch weiter zu erhöhen, ist es eine vorteilhafte Massnahme, die erste Kühlleitung 8 und/oder die zweite Kühlleitung 9 zu erden. Dies kann beispielsweise mit einem Erdungskabel realisiert werden, das einerseits mit der ersten Kühlleitung 8 und/oder mit der zweiten Kühlleitung 9 verbunden ist und andererseits mit dem Erdpotential.

Die Innenraumschleife 90 kann auch in einer anderen Form ausgestaltet sein als die vorangehend beschriebenen Kühlspirale 91. Die Innenraumschleife 90 kann beispielsweise mäandrierend oder wellenförmig ausgestaltet sein, wobei z.B. die Wellenberge benachbart zu den zweiten Enden der Längsschenkel 26 angeordnet sind, und die Wellentäler benachbart zu den ersten Enden der Längsschenkel 26.

Fig. 5 zeigt eine perspektivische Darstellung eines dritten Ausführungsbeispiels eines erfindungsgemässen Drehantriebs 1. In Fig. 5 ist ein Segment aus dem Stator 2 herausgeschnitten, damit das Innere des von den Spulenkernen 25 umgebenen Raums erkennbar ist. Zum besseren Verständnis zeigt Fig. 6 in einer perspektivischen Darstellung die Kühlvorrichtung 10 des dritten Ausführungsbeispiels.

Im Folgenden wird nur auf die Unterschiede zu dem ersten und dem zweiten Ausführungsbeispiel eingegangen. Gleiche Teile oder von der Funktion her gleichwertige Teile des dritten Ausführungsbeispiels sind mit den gleichen Bezugszeichen bezeichnet wie bei dem ersten und dem zweiten Ausführungsbeispiel. Insbesondere haben die Bezugszeichen die gleiche Bedeutung wie sie bereits im Zusammenhang mit dem ersten und dem zweiten Ausführungsbeispiel erläutert sind. Es versteht sich, dass alle vorangehenden Erläuterungen des ersten und des zweiten Ausführungsbeispiels in gleicher Weise oder in sinngemäss gleicher Weise auch für das dritte Ausführungsbeispiel gelten.

Bei dem dritten Ausführungsbeispiel umfasst die Kühlvorrichtung 10 eine erste Ringleitung 93, die von dem Kühlfluid durchströmbar ist, wobei sich die erste Ringleitung 93 in der Umfangsrichtung radial innenliegend entlang der Längsschenkel 26 der Spulenkerne 25 erstreckt, und wobei die erste Ringleitung 93 bezüglich der axialen Richtung A zwischen der ersten Platine 71 und den Querschenkeln 27 angeordnet ist. Darstellungsgemäss (Fig. 5) ist die erste Ringleitung 93 also oberhalb der ersten Platine 71 angeordnet.

Die erste Ringleitung 93 ist im Wesentlichen kreisförmig ausgestaltet und verläuft in einer Ebene, die parallel zur radialen Ebene E ist. Mit der ersten Ringleitung 93 lässt sich insbesondere Wärme aus demjenigen Bereich abführen, in welchem die erste Platine 71 mit ihren elektronischen Komponenten angeordnet ist. Dies Wärme entsteht beispielsweise beim Betrieb des Drehantriebs 1 durch das Fliessen von elektrischen Strömen in der ersten Platine 71 und durch die Kupfer- und Eisenverluste im Drehantrieb 1 .

Ferner wird bei solchen Anwendungen, bei denen mit dem Drehantrieb 1 Fluide mit sehr hoher Temperatur gefördert oder gemischt werden zusätzlich von dem Fluid noch Wärme in den elektromagnetischen Drehantrieb eingetragen. Da sich die erste Platine 71 in der Nachbarschaft des Rotors 3 befindet, kann beim Fördern oder Mischen sehr heisser Fluide ein beträchtlicher Wärmeeintrag von dem Fluid in die erste Platine 71 verursacht werden. Bei solchen Anwendungen ist die erste Ringleitung 93 besonders vorteilhaft, weil mit ihr gezielt die Wärme aus dem Bereich abführbar ist, in welchem die erste Platine 71 angeordnet ist.

Vorzugsweise ist die erste Ringleitung 93 integraler Bestandteil der zweiten Kühlleitung und in Serie mit der Kühlspirale 91 angeordnet. Im Unterschied zum zweiten Ausführungsbeispiel umfasst also bei dem dritten Ausführungsbeispiel die zweite Kühlleitung 9 zusätzlich die erste Ringleitung 93.

Vorzugsweise ist die erste Ringleitung 93 strömungstechnisch gesehen zwischen dem ersten Ende 911 der Kühlspirale 91 und dem zweiten Kühlanschluss 12 angeordnet. Der zweite Kühlanschluss 12 ist über ein Verbindungssegment 92 der zweiten Kühlleitung 9 mit einem Ende der ersten Ringleitung 93 verbunden. Das andere Ende der ersten Ringleitung 93 ist über ein weiteres Verbindungssegment 92 der zweiten Kühlleitung 9 mit dem ersten Ende 911 der Kühlspirale 9 verbunden.

Im Betriebszustand wird das Kühlfluid durch einen der beiden Kühlanschlüsse 11, 12 beispielsweise durch den ersten Kühlanschluss 11 zugeführt, durchströmt die erste Kühlleitung 8 mit den Kühlschleifen 81, strömt dann durch das Verbindungselement 98 in die zweite Kühlleitung 9, durchströmt die Kühlspirale 91, anschliessend die erste Ringleitung 93 und fliesst nach Durchströmen der zweite Kühlleitung 9 durch den zweiten Kühlanschluss 12 ab.

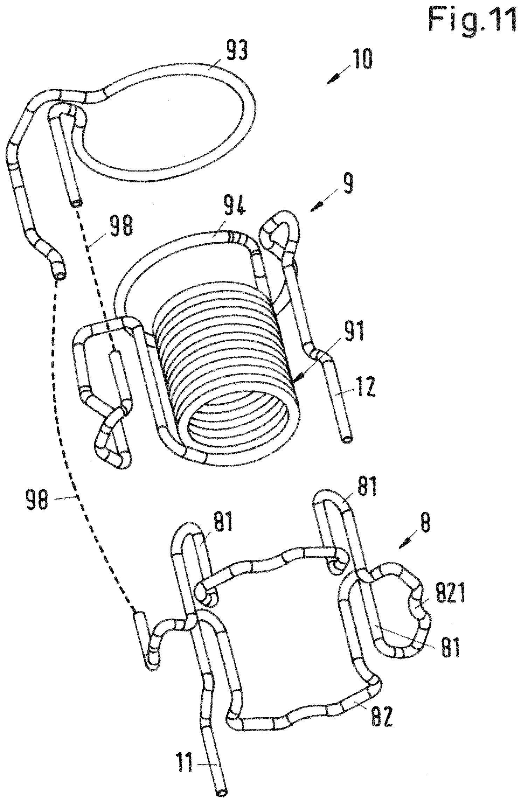

Alternativ oder ergänzend zu der ersten Ringleitung 93 kann auch eine zweite Ringleitung 94 (Fig. 11) vorgesehen sein, die von dem Kühlfluid durchströmbar ist, wobei sich die zweite Ringleitung 94 auch in der Umfangsrichtung radial innenliegend entlang der Längsschenkel 26 der Spulenkerne 25 erstreckt, und wobei die zweite Ringleitung 94 bezüglich der axialen Richtung zwischen der ersten Platine 71 und den Wicklungen 61b angeordnet ist. Die zweite Ringleitung 94 ist in Fig. 5 nicht dargestellt, kann aber in sinngemäss gleicher Weise ausgestaltet und angeordnet sein, wie dies im Zusammenhang mit Fig. 11 noch erläutert wird.

Gemäss der Darstellung in Fig. 5 ist die zweite Ringleitung 94 (nicht dargestellt) dann also unterhalb der ersten Platine 71 und unmittelbar benachbart zu der ersten Platine 71 angeordnet. Die zweite Ringleitung 94 ist im Wesentlichen kreisförmig ausgestaltet und verläuft in einer Ebene, die parallel zur radialen Ebene E ist.

Vorzugsweise ist die zweite Ringleitung 94 integraler Bestandteil der zweiten Kühlleitung 9 und in Serie mit der Kühlspirale 91 angeordnet.

Es sind Ausgestaltungen möglich, bei denen entweder die erste Ringleitung 93 oder die zweite Ringleitung 94 vorgesehen ist, es sind aber auch Ausgestaltungen möglich, bei denen die Kühlvorrichtung 10 die erste Ringleitung 93 und die zweite Ringleitung 94 aufweist.

Fig. 7 zeigt eine Variante des dritten Ausführungsbeispiels in einer perspektivischen Darstellung wie Fig. 5. Bei dieser Variante wird die Antriebs- und Lagerfunktion mit nur einem einzigen Wicklungssystem, nämlich mit den konzentrierten Wicklungen 61 generiert, sodass es also keine Unterscheidung zwischen Antriebs- und Steuerspulen gibt.

Das Wicklungssystem ist so ausgestaltet, dass um jeden Längsschenkel 26 herum jeweils genau eine konzentrierte Wicklung 61 gewickelt ist. Mit diesen konzentrierten Wicklungen 61 werden dann im Betriebszustand diejenigen elektromagnetischen Drehfelder erzeugt, mit welchen ein Drehmoment auf den Rotor 3 bewirkt wird, und mit welchen eine beliebig einstellbare Querkraft in radialer Richtung auf den Rotor 3 ausübbar ist, sodass die radiale Position des Rotors 3, also seine Position in der zur axialen Richtung A senkrechten radialen Ebene E, aktiv steuerbar bzw. regelbar ist. Dies kann so realisiert werden, dass die von einer Kontrolleinrichtung jeweils ermittelten Werte für den Antriebs- und den Steuerstrom rechnerisch -also z. B. mit Hilfe von Software - addiert bzw. überlagert werden und der sich daraus ergebende Gesamtstrom in die jeweilige konzentrierte Wicklung 61 eingeprägt wird. In diesem Fall ist es natürlich nicht mehr möglich, zwischen Steuer- und Antriebsspulen zu unterscheiden. In der in Fig. 7 gezeigten Variante gibt es im Stator 2 keine Unterscheidung zwischen Antriebs- und Steuerspulen, sondern es gibt jeweils nur ein Wicklungssystem, in dessen sechs konzentrierten Wicklungen 61 die rechnerisch ermittelte Summe aus dem Antriebs- und dem Steuerstrom eingeprägt wird.

Es versteht sich, dass auch das erste Ausführungsbeispiel (Fig. 1) und das zweite Ausführungsbeispiel (Fig. 3) in analoger Weise wie die in Fig. 7 dargestellte Variante ausgestaltet sein können, also mit genau einer konzentrierten Wicklung 61 an jedem Längsschenkel 26 anstelle der separaten Antriebsspulen 61a und der separaten Steuerspulen 61b.

Fig. 8 zeigt eine perspektivische Darstellung eines vierten Ausführungsbeispiels eines erfindungsgemässen Drehantriebs 1. Zum besseren Verständnis zeigt Fig. 9 in einer perspektivischen Darstellung die Kühlvorrichtung 10 des vierten Ausführungsbeispiels.

Im Folgenden wird nur auf die Unterschiede zu den vorangehend beschriebenen Ausführungsbeispielen eingegangen. Gleiche Teile oder von der Funktion her gleichwertige Teile des vierten Ausführungsbeispiels sind mit den gleichen Bezugszeichen bezeichnet wie bei den vorangehend beschriebenen Ausführungsbeispielen. Insbesondere haben die Bezugszeichen die gleiche Bedeutung wie sie bereits im Zusammenhang mit den vorangehend beschriebenen Ausführungsbeispielen und Varianten erläutert sind. Es versteht sich, dass alle vorangehenden Erläuterungen der Ausführungsbeispiele und Varianten in gleicher Weise oder in sinngemäss gleicher Weise auch für das vierte Ausführungsbeispiel gelten.

Bei dem vierten Ausführungsbeispiel umfasst die Kühlvorrichtung 10 eine dritte Ringleitung 83, welche von dem Kühlfluid durchströmbar ist, und welche primär zur Kühlung einer zweiten Platine 72 dient, die elektronische Komponenten aufweist. Die elektronischen Komponenten sind in Fig. 8 nicht dargestellt. Die zweite Platine 72 ist vorzugsweise als Elektronikprint bzw. PCB (printed circuit board) ausgestaltet.

Die zweite Platine 72 ist im Wesentlichen scheibenförmig ausgestaltet und parallel zur radialen Ebene E angeordnet. Bezüglich der axialen Richtung A ist die zweite Platine 72 benachbart zu den ersten Enden der Längsschenkel 26 auf der den Querschenkeln 27 abgewandten Seite der ersten Enden angeordnet. Gemäss der Darstellung in Fig. 8 ist die zweite Platine unterhalb der ersten Enden der Längsschenkel 26 und somit auch unterhalb des Rückschlusses 22.

Auf der zweiten Platine 72 kann beispielsweise die Kontrolleinrichtung für den Drehantrieb 1 angeordnet sein, welche die Leistungselektronik zur Ansteuerung der Wicklungen 61 bzw. 61a, 61b und zur Generierung der elektromagnetischen Felder, die Regeleinrichtung für den Antrieb und die Lagerung des Rotors und gegebenenfalls Sensoren oder Auswerteeinheiten umfassen kann Die zweite Platine 72 ist in dem Statorgehäuse 20 angeordnet und wird von dem Statorgehäuse 20 vollständig umschlossen.

Die dritte Ringleitung 83 erstreckt sich in der Umfangsrichtung entlang der ersten Enden der Längsschenkel 26, und ist bezüglich der axialen Richtung A zwischen der zweiten Platine 72 und den ersten Enden der Längsschenkel 26 bzw. dem Rückschluss 22 angeordnet Darstellungsgemäss (Fig. 8) ist die dritte Ringleitung 83 also oberhalb der zweiten Platine 72 angeordnet.

Alternativ oder ergänzend zu der dritten Ringleitung 83 kann auch eine vierte Ringleitung 84 vorgesehen sein, die in Fig. 8 nur mittels einer gestrichelten Linie angedeutet ist. Auch die vierte Ringleitung 84 ist von dem Kühlfluid durchströmbar. Die vierte Ringleitung 84 erstreckt sich in der Umfangsrichtung und ist parallel zur radialen Ebene E angeordnet. Bezüglich der axialen Richtung A ist die vierte Ringleitung 84 derart angeordnet, dass sich die zweite Platine 72 bezüglich der axialen Richtung A zwischen den ersten Enden der Längsschenkel 26 und der vierten Ringleitung 84 befindet. Bezüglich der Darstellung in Fig. 8 ist die vierte Ringleitung 84 unterhalb der zweiten Platine 72 angeordnet.

Es sind Ausgestaltungen möglich, bei denen entweder die dritte Ringleitung 83 oder die vierte Ringleitung 84 vorgesehen ist, es sind aber auch Ausgestaltungen möglich, bei denen die Kühlvorrichtung 10 die dritte Ringleitung 83 und die vierte Ringleitung 84 aufweist.

Die dritte Ringleitung 83 und die vierte Ringleitung sind im Wesentlichen jeweils kreisförmig ausgestaltet und verlaufen jeweils in einer Ebene, die parallel zur radialen Ebene E bzw. parallel zu dem Rückschluss 22 ist.

Vorzugsweise sind die dritte Ringleitung 83 und/oder die vierte Ringleitung 84 integraler Bestandteil der ersten Kühlleitung 8 und jeweils in Serie mit den Kühlschleifen 81 angeordnet. Im Unterschied zu den vorangehend beschriebenen Ausführungsbeispielen umfasst also bei dem vierten Ausführungsbeispiel die erste Kühlleitung 8 zusätzlich die dritte Ringleitung 83 und/oder die vierte Ringleitung 84.

Bei Ausgestaltungen, bei denen sowohl die dritte Ringleitung 83 als auch die vierte Ringleitung 84 vorgesehen sind, sind die dritte Ringleitung 83 und die vierte Ringleitung 84 vorzugsweise in Serie - also hintereinander - angeordnet..

Vorzugsweise sind die dritte Ringleitung 83 und/oder die vierte Ringleitung 84 strömungstechnisch gesehen zwischen dem ersten Kühlanschluss 11 und den Kühlschleifen 81 angeordnet. Der erste Kühlanschluss 11 ist über ein Verbindungssegment 82 der ersten Kühlleitung 8 mit einem Ende der dritten Ringleitung 83 oder der vierten Ringleitung 84 verbunden. Das zweite Ende der dritten Ringleitung 83 ist über ein weiteres Verbindungssegment 82 der ersten Kühlleitung 8 mit einer der Kühlschleifen 81 verbunden.

Im Betriebszustand wird das Kühlfluid durch einen der beiden Kühlanschlüsse 11, 12 beispielsweise durch den ersten Kühlanschluss 11 zugeführt, durchströmt zunächst die vierte Ringleitung 84und/oder die dritte Ringleitung 83, dann die Kühlschleifen 81 der ersten Kühlleitung 8, strömt anschliessend durch das Verbindungselement 98 in die zweite Kühlleitung 9, durchströmt die Kühlspirale 91, und die erste Ringleitung 93, und fliesst nach Durchströmen der zweite Kühlleitung 9 durch den zweiten Kühlanschluss 12 ab.

Fig. 10 zeigt eine perspektivische Darstellung eines fünften Ausführungsbeispiels eines erfindungsgemässen Drehantriebs 1. In Fig. 10 ist ein Segment aus dem Stator 2 herausgeschnitten, damit das Innere des von den Spulenkernen 25 umgebenen Raums erkennbar ist. Fig. 11 zeigt in einer perspektivischen Darstellung die Kühlvorrichtung 10 des fünften Ausführungsbeispiels. Zum besseren Verständnis zeigt Fig. 11 die Kühlvorrichtung 10 in einer Explosionsdarstellung

Im Folgenden wird nur auf die Unterschiede zu den vorangehend beschriebenen Ausführungsbeispielen eingegangen. Gleiche Teile oder von der Funktion her gleichwertige Teile des fünften Ausführungsbeispiels sind mit den gleichen Bezugszeichen bezeichnet wie bei den vorangehend beschriebenen Ausführungsbeispielen. Insbesondere haben die Bezugszeichen die gleiche Bedeutung wie sie bereits im Zusammenhang mit den vorangehend beschriebenen Ausführungsbeispielen und Varianten erläutert sind. Es versteht sich, dass alle vorangehenden Erläuterungen der Ausführungsbeispiele und Varianten in gleicher Weise oder in sinngemäss gleicher Weise auch für das fünfte Ausführungsbeispiel gelten.

Bei dem fünften Ausführungsbeispiel hat der Stator 2 des elektromagnetischen Drehantriebs 1 acht Spulenkerne 25 die kreisförmig und äquidistant um den Rotor 3 herum angeordnet sind. Bei dem fünften Ausführungsbeispiel sind an den Längsschenkeln 26 der Spulenkerne 25 separate Antriebsspulen 61a und separate Steuerspulen 61b vorgesehen.

Der Stator 2 umfasst eine Mehrzahl von konzentrierten Antriebsspulen 61a, hier nämlich vier Antriebsspulen 61a, von denen jede jeweils um die Längsschenkel 26 zweier benachbarter Spulenkerne 25 herum angeordnet ist, sodass diese beiden Längsschenkel 26 bezüglich der radialen Richtung innerhalb der Antriebsspule 61a angeordnet sind. Jede Antriebsspule 61a ist also jeweils um genau zwei Längsschenkel 26 herum gewickelt, sodass beide Längsschenkel 26 im Innenraum dieser Antriebsspule 61a angeordnet sind.

Ferner ist an jedem der Längsschenkel 26 jeweils genau eine Steuerspule 61b vorgesehen, die um den jeweiligen Längsschenkel 26 herum angeordnet ist. Bezüglich der axialen Richtung A sind die Steuerspulen 61b benachbart zu den Antriebsspulen 61a angeordnet, hier nämlich darstellungsgemäss (Fig. 10) oberhalb der Antriebsspulen.

Bei dem fünften Ausführungsbeispiel umfasst die Kühlvorrichtung 10 die erste Kühlleitung 8 und die zweite Kühlleitung 9.

Die erste Kühlleitung 8 umfasst die vier Kühlschleifen 81 sowie die Verbindungssegmente 82. Die Verbindungssegmente 82, welche zwei Kühlschleifen 81 miteinander verbinden, können dabei Einbuchtungen 821 aufweisen, die radial nach innen ausgerichtet und in Umfangsrichtung zwischen den ersten Enden zweier benachbarter Längsschenkel 26 angeordnet sind. Die Einbuchtungen 821 dienen primär dazu, die erste Kühlleitung 8 näher an den Rückschluss 22 heranzuführen, um so die Wärmeabfuhr noch zu verstärken.

Jede der Kühlschleifen 81 ist bezüglich der radialen Richtung innerhalb einer der Antriebsspulen 61a angeordnet. Die Kühlschleifen 81 jeweils im Innenraum einer der Antriebsspulen anzuordnen, hat den Vorteil, dass insbesondere die durch die Kupferverluste generierte Wärme besonders effizient abgeführt werden kann.

Bei dem fünften Ausführungsbeispiel sind auch solche Varianten möglich, bei denen die erste Kühlleitung 8 zusätzlich die dritte Ringleitung 83 zur Kühlung der zweiten Platine 72 umfasst, in sinngemäss gleicher Weise, wie dies für das vierte Ausführungsbeispiel beschrieben wurde.

Die zweite Kühlleitung 9 umfasst die Kühlspirale 91, die erste Ringleitung 93, die bezüglich der axialen Richtung A zwischen der ersten Platine 71 und den Querschenkeln 27 angeordnet ist, sowie die zweite Ringleitung 94, die bezüglich der axialen Richtung A zwischen der ersten Platine 71 und den Wicklungen 61b angeordnet ist. Bei dieser Ausgestaltung umfasst die zweite Kühlleitung 9 also sowohl die erste Ringleitung 93, die darstellungsgemäss (Fig. 10) oberhalb der ersten Platine 71 vorgesehen ist, als auch die zweite Ringleitung 94, die darstellungsgemäss unterhalb der ersten Platine 71 angeordnet ist.

Es versteht sich, dass für das fünfte Ausführungsbeispiel auch solche Varianten möglich sind, bei denen die erste Ringleitung 93, nicht aber die zweite Ringleitung 94 vorgesehen ist, oder solche Varianten, bei denen die zweite Ringleitung 94, nicht aber die erste Ringleitung 93 vorgesehen ist oder solche Varianten, bei denen die zweite Kühlleitung 9 weder die erste Ringleitung 93 noch die zweite Ringleitung 94 aufweist.

Bei der Kühlvorrichtung 10 ist die erste Kühlleitung 8 vorzugsweise einstückig ausgestaltet. Aus konstruktiven Gründen kann es vorteilhaft sein, die zweite Kühlleitung 9 - wie in Fig. 11 gezeigt - aus zwei Einzelstücken zusammenzusetzen, wobei das eine Einzelstück die Kühlspirale 91 sowie die zweite Ringleitung 94 umfasst, und das andere Einzelstück die erste Ringleitung 93. Diese beiden Einzelstücke können dann über ein Verbindungselement 98 miteinander verbunden werden, wobei das Verbindungselement in analoger Weise ausgestaltet sein kann, wie dies im Zusammenhang mit dem zweiten Ausführungsbeispiel (Fig. 3, Fig. 4) erläutert ist.

Die zweite Kühlleitung 9 ist wiederum mit der ersten Kühlleitung 8 über das Verbindungselement 98 verbunden. Aufgrund der Explosionsdarstellung in Fig. 11 sind dort die Verbindungselemente 98 jeweils nur durch die gestrichelten Linien mit dem Bezugszeichen 98 angedeutet.

Im Betriebszustand wird das Kühlfluid durch einen der beiden Kühlanschlüsse 11, 12 beispielsweise durch den ersten Kühlanschluss 11 zugeführt, durchströmt zunächst die Kühlschleifen 81 der ersten Kühlleitung 8, strömt anschliessend durch das Verbindungselement 98 in die zweite Kühlleitung 9, durchströmt die erste Ringleitung 93, dann die Kühlspirale 91und die zweite Ringleitung 94, und fliesst nach Durchströmen der zweite Kühlleitung 9 durch den zweiten Kühlanschluss 12 ab.

In der zweiten Kühlleitung ist somit strömungstechnisch gesehen die Kühlspirale 91 zwischen der ersten Ringleitung 93 und der zweiten Ringleitung 94 angeordnet.

Durch die Erfindung wird ferner eine Zentrifugalpumpe 100 zum Fördern eines Fluids vorgeschlagen, die dadurch gekennzeichnet ist, dass die Zentrifugalpumpe 100 einen elektromagnetischen Drehantrieb 1 umfasst, der erfindungsgemäss ausgestaltet ist, wobei der Rotor 3 des elektromagnetischen Drehantriebs 1 als Rotor 3 der Zentrifugalpumpe 100 ausgestaltet ist.

Fig. 12 zeigt ein Ausführungsbeispiel einer erfindungsgemässen Zentrifugalpumpe, die gesamthaft mit dem Bezugszeichen 100 bezeichnet ist, in einer perspektivischen Darstellung. Zum besseren Verständnis zeigt Fig. 13 eine schematische Schnittdarstellung des Ausführungsbeispiels aus Fig. 12 in einem Schnitt in axialer Richtung A. In Fig. 13 sind zum besseren Verständnis das Statorgehäuse 20 und die Kühlvorrichtung 11 nicht dargestellt.

Die Zentrifugalpumpe 100 umfasst eine Pumpeneinheit 50 mit einem Pumpengehäuse 51, das einen Einlass 52 und einen Auslass 53 für das zu fördernde Fluid umfasst, wobei der Rotor 3 im Pumpengehäuse 51 angeordnet ist, und eine Mehrzahl von Flügeln 54 zum Fördern des Fluids umfasst. Die Pumpeneinheit 50 ist derart ausgestaltet, dass die Pumpeneinheit 50 so in den Stator 2 einsetzbar ist, dass der magnetisch wirksame Kern 31 des Rotors 3 von den Stirnflächen 211 der Querschenkel 27 umgeben wird.

Ein vorteilhafter Aspekt ist es, dass der Rotor 3 als Integralrotor ausgestaltet ist, weil er sowohl der Rotor 3 des elektromagnetischen Drehantriebs 1 ist als auch der Rotor 3 der Zentrifugalpumpe 100, mit welchem das Fluid gefördert wird. Insgesamt erfüllt der Rotor 3 somit drei Funktionen in einem: Er ist der Rotor 3 des elektromagnetischen Antriebs 1, er ist der Rotor 3 der magnetischen Lagerung, und er ist das Laufrad, mit welchem auf das Fluid bzw. die Fluide eingewirkt wird. Diese Ausgestaltung als Integralrotor bietet den Vorteil einer sehr kompakten und platzsparenden Ausgestaltung.

Der Stator 2 ist in dem Statorgehäuse 20 angeordnet, das vorzugsweise als hermetisch dichtes Statorgehäuse 20 ausgestaltet ist und den Stator 2 umkapselt. Falls der Drehantrieb 1 mit der ersten Platine 71 ausgestaltet ist, so ist diese auch in dem Statorgehäuse 20 angeordnet. Falls der Drehantrieb 1 mit der zweiten Platine 72 ausgestaltet ist, so ist diese vorzugsweise, aber nicht notwendigerweise auch in dem Statorgehäuse 20 angeordnet. Das Statorgehäuse 20 ist vorzugsweise mit einer Vergussmasse, beispielsweise mit einem Epoxidharz oder mit einem Polyurethan, aufgefüllt, sodass der Stator 2 und gegebenenfalls weitere Komponenten, die im Inneren des Statorgehäuses 20 angeordnet sind, von der Vergussmasse umgeben sind.

Das Statorgehäuse 20 weist zudem an seinem der Pumpeneinheit 50 zugewandten Ende eine Ausnehmung auf, sodass die Pumpeneinheit 50 in diese Ausnehmung einsetzbar ist. Der in dem Pumpengehäuse 51 vorgesehene Rotor 3 wird dann von dieser Ausnehmung im Statorgehäuse 20 umschlossen, wobei der magnetisch wirksame Kern 31 des Rotors 3 zwischen den Querschenkeln 27 der Spulenkerne 26 angeordnet ist.

Das Pumpengehäuse 51 ist am Statorgehäuse 20 fixiert, vorzugsweise mit einer Mehrzahl von Schrauben 511.

Die Kühlanschlüsse 11, 12 sind vorzugsweise an der der Pumpeneinheit 50 abgewandten Seite des Statorgehäuses 20 vorgesehen und daher in Fig. 12 nicht erkennbar.

Der Rotor 3 umfasst die Mehrzahl von Flügeln 54 zum Fördern des Fluids. Bei dem hier beschriebenen Ausführungsbeispiel sind beispielsweise insgesamt vier Flügel 54 vorgesehen, wobei diese Anzahl beispielhaften Charakter hat. Der Rotor 3 umfasst ferner eine Ummantelung 38, mit welcher der magnetisch wirksame Kern 31 des Rotors 3 umschlossen und vorzugsweise hermetisch eingekapselt ist, sodass der magnetisch wirksame Kern 31 des Rotors 3 nicht in Kontakt mit dem zu fördernden Fluid kommt. Alle Flügel 54 sind auf der Ummantelung 38 angeordnet und bezüglich der Umfangsrichtung des Rotors 3 äquidistant angeordnet. Jeder Flügel 54 erstreckt sich in radialer Richtung nach aussen und ist drehfest mit dem Ummantelung 38 verbunden. Die Flügel 54 können separate Komponenten sein, die dann auf der Ummantelung 38 fixiert werden. Es ist natürlich auch möglich, dass alle Flügel 54 integraler Bestandteil der Ummantelung 38 sind, dass also die Ummantelung 38 mit allen Flügeln 54 einstückig ausgestaltet ist. Der Rotor 3 mit den Flügeln 54 bildet das Flügelrad bzw. das Laufrad der Zentrifugalpumpe 100, mit welchem auf das Fluid oder die Fluide eingewirkt wird.

Je nach Anwendungsfall, ist es bevorzugt, wenn das Pumpengehäuse 51 der Pumpeneinheit 50 sowie die Ummantelung 38 und die Flügel 54 aus einem oder mehreren Kunststoffen hergestellt werden. Geeignete Kunststoffe sind: Polyethylene (PE), Low Density Polyethylene (LDPE), Ultra Low Density Polyethylene (ULDPE), Ethylene Vinyl Acetate (EVA), Polyethylene Terephthalate (PET), Polyvinylchlorid (PVC), Polypropylene (PP), Polyurethan (PU), Polyvinylidene Fluoride (PVDF), Acrylonitrile Butadiene Styrene (ABS), Polyacryl, Polycarbonate (PC), Polyetheretherketon (PEEK) oder Silicone. Für viele Anwendungen sind auch die unter dem Markennamen Teflon bekannten Materialien Polytetrafluoroethylene (PTFE) und und Perfluoralkoxy-Polymere (PFA) als Kunststoff geeignet.

Es versteht sich, dass der erfindungsgemässe elektromagnetische Drehantrieb auch für andere Vorrichtungen als Zentrifugalpumpen geeignet ist, beispielsweise für Mischvorrichtungen zum Mischen fliessfähiger Substanzen, für Rührvorrichtungen, beispielsweise zum Durchmischen eines Fluids in einem Tank, für Lüfter oder auch für Vorrichtungen zum Tragen und Rotieren von Wafern, beispielsweise in der Halbleiterfertigung.

## Patentansprüche

1. Elektromagnetischer Drehantrieb, der als Tempelmotor ausgestaltet ist, mit einem Rotor (3), der einen ring- oder scheibenförmigen magnetisch wirksamen Kern (31) umfasst, sowie mit einem Stator (2), welcher als Lager- und Antriebsstator ausgestaltet ist, mit welchem der Rotor (3) im Betriebszustand berührungslos magnetisch um eine Solldrehachse antreibbar ist, die eine axiale Richtung (A) definiert, und mit welchem der Rotor (3) berührungslos magnetisch bezüglich des Stators (2) lagerbar ist, wobei der Rotor (3) in einer zur axialen Richtung (A) senkrechten radialen Ebene (E) aktiv magnetisch gelagert ist, wobei der Stator (2) eine Mehrzahl von Spulenkernen (25) aufweist, von denen jeder einen Längsschenkel (26) umfasst, welcher sich von einem ersten Ende in axialer Richtung (A) bis zu einem zweiten Ende erstreckt, sowie einen Querschenkel (27), welcher an dem zweiten Ende des Längsschenkels (26) und in der radialen Ebene (E) angeordnet ist, und welcher sich von dem Längsschenkel (26) in einer radialen Richtung erstreckt, wobei die Spulenkerne (25) bezüglich einer Umfangsrichtung um den magnetisch wirksamen Kern (31) herum angeordnet sind, und wobei an jedem Längsschenkel (26) mindestens eine konzentrierte Wicklung (61; 61a, 61b) vorgesehen ist, welche den jeweiligen Längsschenkel (26) umgibt, **dadurch gekennzeichnet, dass** eine Kühlvorrichtung (10) vorgesehen ist, welche eine erste Kühlleitung (8) umfasst, die von einem Kühlfluid durchströmbar ist, wobei die erste Kühlleitung (8) mindestens einen Kühlleitungsabschnitt (80) aufweist, welcher bezüglich der Umfangsrichtung in einem Zwischenraum zwischen den Längsschenkeln (26) zweier benachbarter Spulenkerne (25) angeordnet ist, und welcher sich in der axialen Richtung (A) erstreckt.

2. Elektromagnetischer Drehantrieb nach Anspruch 1, wobei die erste Kühlleitung (8) mindestens eine Kühlschleife (81) und vorzugsweise mehrere Kühlschleifen (81) aufweist, wobei jede Kühlschleife (81) bezüglich der Umfangsrichtung jeweils in einem Zwischenraum zwischen den Längsschenkeln (26) zweier benachbarter Spulenkerne (25) angeordnet ist, und wobei jeder Kühlleitungsabschnitt (80) jeweils Bestandteil einer der Kühlschleifen (81) ist.

3. Elektromagnetischer Drehantrieb nach Anspruch 2, wobei die erste Kühlleitung (8) mehrere Kühlschleifen (81) aufweist, sowie Verbindungssegmente (82) zum Verbinden benachbarter Kühlschleifen (81), wobei sich die Verbindungssegmente (82) jeweils in der Umfangsrichtung erstrecken, und radial aussenliegend benachbart zu den ersten Enden der Längsschenkel (26) der Spulenkerne (25) angeordnet sind.

4. Elektromagnetischer Drehantrieb nach einem der vorangehenden Ansprüche, bei welchem die ersten Enden aller Längsschenkel (26) mittels eines Rückschlusses (22) zum Führen des magnetischen Flusses verbunden sind, wobei die erste Kühlleitung (8) bereichsweise benachbart zu dem Rückschluss (22) verläuft.

5. Elektromagnetischer Drehantrieb nach einem der vorangehenden Ansprüche, wobei die Kühlvorrichtung (10) eine zweite Kühlleitung (9) umfasst, die von dem Kühlfluid durchströmbar ist, wobei die zweite Kühlleitung (9) eine Innenraumschleife (90) aufweist, welche radial innenliegend bezüglich der Wicklungen (61; 61a, 61b) in dem von den Wicklungen (61; 61a, 61b) umgebenen Innenraum angeordnet ist, wobei die Innenraumschleife (90) vorzugsweise als Kühlspirale (91) ausgestaltet ist.

6. Elektromagnetischer Drehantrieb nach Anspruch 5, wobei die erste Kühlleitung (8) und die zweite Kühlleitung (9) in Serie miteinander verbunden sind.

7. Elektromagnetischer Drehantrieb nach einem der vorangehenden Ansprüche, bei welchem bezüglich der axialen Richtung (A) zwischen den Wicklungen (61; 61b) und den Querschenkeln (27) eine erste Platine (71) mit elektronischen Komponenten angeordnet ist, und bei welchem die Kühlvorrichtung (10) eine erste Ringleitung (93) oder eine zweite Ringleitung (94) umfasst, die jeweils von dem Kühlfluid durchströmbar sind, wobei sich die erste Ringleitung (93) und die zweite Ringleitung (94) jeweils in der Umfangsrichtung radial innenliegend entlang der Längsschenkel (26) der Spulenkerne (25) erstrecken, wobei die erste Ringleitung (93) bezüglich der axialen Richtung (A) zwischen der ersten Platine (71) und den Querschenkeln (27) angeordnet ist, und wobei die zweite Ringleitung (94) bezüglich der axialen Richtung (A) zwischen der ersten Platine (71) und den Wicklungen (61; 61b) angeordnet ist.

8. Elektromagnetischer Drehantrieb nach Anspruch 7, wobei die Kühlvorrichtung (10) die erste Ringleitung (93) und die zweite Ringleitung (94) aufweist.

9. Elektromagnetischer Drehantrieb nach einem der Ansprüche 7-8, wobei die erste Ringleitung (93) und/oder die zweite Ringleitung (94) integraler Bestandteil der zweiten Kühlleitung (9) sind, und in Serie mit der Innenraumschleife (90) bzw. der Kühlspirale (91) verbunden sind.

10. Elektromagnetischer Drehantrieb nach einem der vorangehenden Ansprüche, bei welchem eine zweite Platine (72) mit elektronischen Komponenten vorgesehen ist, die bezüglich der axialen Richtung (A) benachbart zu den ersten Enden der Längsschenkel (26) auf der den Querschenkeln (27) abgewandten Seite angeordnet ist, und bei welchem die Kühlvorrichtung (10) eine dritte Ringleitung (83) oder eine vierte Ringleitung (84) umfasst, die jeweils von dem Kühlfluid durchströmbar sind, wobei sich die dritte Ringleitung (83) und die vierte Ringleitung (84) jeweils in der Umfangsrichtung erstrecken, wobei die dritte Ringleitung (83) bezüglich der axialen Richtung (A) zwischen der zweiten Platine (72) und den ersten Enden der Längsschenkel (26) angeordnet ist, und wobei die vierte Ringleitung (84) derart angeordnet ist, dass sich die zweite Platine (72) bezüglich der axialen Richtung (A) zwischen den ersten Enden der Längsschenkel (26) und der vierten Ringleitung (84) befindet.

11. Elektromagnetischer Drehantrieb nach Anspruch 10, wobei die dritte Ringleitung (83) und/oder die vierte Ringleitung (84) integraler Bestandteil der ersten Kühlleitung (8) sind, und in Serie mit der mindestens einen Kühlschleife (81) verbunden sind.

12. Elektromagnetischer Drehantrieb nach einem der vorangehenden Ansprüche, wobei an jedem Längsschenkel (26) zwei konzentrierte Wicklungen (61a, 61b) vorgesehen sind, von denen jede den jeweiligen Längsschenkel (26) umgibt, und die bezüglich der axialen Richtung (A) benachbart zueinander angeordnet sind.

13. Elektromagnetischer Drehantrieb nach einem der Ansprüche 1-11, wobei eine Mehrzahl von konzentrierten Antriebsspulen (61a) vorgesehen ist, von denen jede jeweils um die Längsschenkel (26) zweier benachbarter Spulenkerne (25) herum angeordnet ist, sodass die beiden Längsschenkel (26) bezüglich der radialen Richtung innerhalb der Antriebsspule (61a) angeordnet sind.

14. Elektromagnetischer Drehantrieb nach Anspruch 13, wobei mindestens einer der Kühlleitungsabschnitte (80) oder eine der Kühlschleifen (81) bezüglich der radialen Richtung innerhalb einer der Antriebsspulen (61a) angeordnet ist.

15. Zentrifugalpumpe zum Fördern eines Fluids, **dadurch gekennzeichnet, dass** die Zentrifugalpumpe einen elektromagnetischen Drehantrieb (1) umfasst, der gemäss einem der vorangehenden Ansprüche ausgestaltet ist, wobei der Rotor (3) des elektromagnetischen Drehantriebs (1) als Rotor (3) der Zentrifugalpumpe ausgestaltet ist.
